# EUROPEAN PATENT SPECIFICATION

(11) **EP 3 104 867 B1**
(45) Date of publication and mention of the grant of the patent: **19.01.2022**
(21) Application number: 15748491.6
(22) Date of filing: 12.02.2015
(51) Int. Cl.: A61K 31/195, A61K 9/00, A61K 35/28, A61P 35/00

(54) **COMPOSITIONS AND METHODS TO IMPROVE THE HOMING AND GRAFTING OF HEMATOPOETIC STEM CELLS**
ZUSAMMENSETZUNGEN UND VERFAHREN ZUR VERBESSERUNG DES HOMINGS UND DER PFROPFUNG VON HÄMATOPOETISCHEN STAMMZELLEN
COMPOSITIONS ET PROCÉDÉS D'AMÉLIORATION DE LA NOSTOCYTOSE ET LA PRISE DE GREFFE DE CELLULES SOUCHES HÉMATOPOÏÉTIQUES

(30) Priority: 12.02.2014 US 201461938988 P
(43) Date of publication of application: 21.12.2016
(73) Proprietor: 7 Hills Pharma LLC, Houston, TX 77021 (US)
(72) Inventor: MARATHI, Upendra K., Houston, TX 77030 (US)
(74) Representative: Gibbs, Richard
(86) International application number: PCT/US2015/015679
(87) International publication number: WO 2015/123447

(56) References cited:
- WO-A1-2013/082243
- WO-A2-2012/030685
- WO-A2-2012/068251
- WO-A2-2012/068251
- KR-B1- 101 000 358
- US-A1- 2013 344 038
- QUESENBERRY, PETER J. ET AL.: 'Stem cell homing: Rolling, crawling, and nesting' PROC. NATL. ACAD. SCI. vol. 95, no. 26, December 1998, pages 15155 - 15157, XP055219531
- VANDERSLICE, PETER ET AL.: 'Small Molecule Agonist of Very Late Antigen-4 (VLA-4) Integrin Induces Progenitor Cell Adhesion' THE JOURNAL OF BIOLOGICAL CHEMISTRY vol. 288, no. 27, 05 July 2013, pages 19414 - 19428, XP055103270
- HERTL,MARTIN ET AL.: 'Hematopoietic Stem Cell Transplantation' MERCK MANUALS PROFESSIONAL VERSION, [Online] April 2013, XP055219532 Retrieved from the Internet: <URL:http://www.merckmanuals.com/profession al/immunology-allergic-disorders/ transplantation/hematopoiet ic-stem- cell -transplantation>

## Description

### BACKGROUND OF THE INVENTION

### 1. Field of the Invention

Embodiments of the present invention relate to composition and methods to improve the homing and grafting of hematopoietic stem cells (HSCs).

More particularly, Embodiments of the present invention relate to compositions and methods to improve the homing and grafting of hematopoietic stem cells (HSCs) into bone marrow, where the methods include *ex vivo* treating HSCs with one or a plurality of selective small molecule agonists or binding agents of very late antigen-4 (VLA-4) integrin.

### 2. Description of the Related Art

The goal of regenerative medicine is to maintain, improve or even restore the function of damaged or diseased cells, tissues, and organs. One way that regenerative medicine aims to revolutionize the practice of medicine is to employ cell-based therapeutics to treat patients. However, for the promise of cell-based therapeutics to be fully realized, the therapeutic cells should be well-tolerated when introduced into a patient, the cells should also migrate or "home" to sites where therapy is needed, and the cells should be capable of providing the therapy desired. The art has attempted to employ stem cell- and progenitor cell-based therapeutics but has met with little, if any, success in a human clinical setting.

One area of regenerative medicine that would benefit from improved cell- based therapeutics are stem cell transplants, *e.g*., bone marrow transplants and hematopoietic stem cell transplants to treat various genetic diseases, cancers, and degenerative disorders. According to the National Marrow Donor Program7 (NMDP), an estimated 45,000 to 50,000 hematopoietic cell transplants are performed annually worldwide to treat patients with life- threatening malignant and non-malignant diseases. However, bone marrow transplantation has numerous drawbacks: bone marrow donation is painful, at times it is difficult and time consuming, and often not possible, to find HLA donor matched tissue; and allogeneic transplants are associated with a significant incidence of graft- versus-host-disease (GVHD). Moreover, although allogeneic hematopoietic stem cell transplants have been performed using more easily obtainable umbilical cord blood, cord blood transplants still have a risk of GVHD. Other drawbacks to existing methods of cord blood transplants, include fewer numbers of transplantable cells and deficient homing and engraftment of donor cells, both of which put the patient at high risk for life threatening infections. In addition, cord blood transplants generally have all the same risks as marrow and peripheral blood transplants.

Numerous approaches have been tried to expand the number of human hematopoietic stem and progenitor cells in cord blood within isolated grafts in ex vivo settings, to reduce the incidence of GVHD, or to increase the ability of the cells to home and engraft, but these efforts have had limited success.

PCT/US2012/066987 published patent application disclosed improved methods for cell therapy. In particular, the invention provides therapeutic compositions of enhanced hematopoietic stem and progenitor cells having improved engrafting and homing properties, and methods of making the therapeutic compositions comprising prostaglandin E2 and derivatives. The invention further provides methods of improving the efficacy of hematopoietic stem and progenitor cell transplantation including transplanting the therapeutic composition to subjects in need of hematopoietic system reconstitution. However, methods employed therein result in a therapeutic cell that has been extensively modified where the expression of variety of cells surface and other signaling molecules are dramatically altered, which raise safety and efficacy concerns. As such, a simple non-pharmacologic approach to improve the homing and grafting of hematopoietic stem and progenitor cells, where the resultant cell is minimally manipulated, is needed.

United States Published Patent Application No. 20130236434A1 disclosed a method of enhancing binding of cells to an integrin-binding ligand comprises treating integrin-expressing cells *in vitro* with an agonist of integrin, wherein the integrin is selected from the group consisting of α4β1 (β-1 integrin, very late antigen 4 (VLA-4)) and contacting the treated cells with an integrin-binding ligand; integrin agonist compounds. The focus of the application is to improve the homing and grafting of endothelial progenitor cells for tissue repair and neovascularization using compounds that intended as candidates for active pharmaceutical ingredients for cardiovascular indications. As such, the final resultant cells after ex vivo treatment are considered to be significantly modified, and thus subject to regulation pre-market review by the FDA. Although the application discloses injection of an *ex vivo* treated integrin expressing cells such as HSCs directly into or around the injured bone marrow, the application does not disclose the direct injection of HSCs treated *ex vivo* with a VLA-4 agonist, and prior to infusing, in a medium or carrier, of cells that are substantially free of VLA-4 agonist, yet retains the ability to graft into bone marrow by intravenous, intra-arterial, and/or intraosseous injection. The advantage of treating and then removing the agonist prior to the introduction of treated cells is that the systemic exposure of the agonist is minimized. As such, any associated toxicity is minimized. By using the compounds to stabilize a pre-existing molecular interaction between an endogenous ligand and receptor, as is the case for divalent cations, the compounds used in the production of the cell therapeutic are considered as an inactive ingredients and/or excipients in the final drug product.

An unrecognized feature of the small molecules in the application and in Vanderslice et al (The Journal of Biological Chemistry, 288, (27), p 19414B 19428, 2013) is that the small molecules function as a preservative, in active ingredient or an excipient in stabilizing normal cell-cell or cell stromal interactions or preserving the adhesive potential mediated by VLA4 and VCAM-1. As such, these socalled agonists may be used as inactive ingredients and/or excipients in the production of medical devices or cell carriers, and the use of medical devices or cell carriers to stabilize and preserve hematopoietic cell-cell interactions ex vivo and/or potential in situ cell-cell interaction after infusion and subsequent suffusion of such cells into bone marrow. As the MnCl₂ synergistically mediates VLA-4 and VCAM-1 interactions, and the small molecule agonist is displaced by VLA-4 increasing the endogenous ligand/receptor interaction from a low affinity state to a higher affinity state, the initial treatment in the presence of Mn⁺⁺ and subsequent removal of the small molecule, with final resultant media, that is essentially free of the small molecule and/or in the presence of about 1mM MnCl₂, carrying the HSC with stabilized, preserved VLA-4 receptor in the high affinity state may mediate sufficient homing and grafting. The application disclosed preferred concentration in a tissue culture media; "In most applications the agonist compound in present in the treatment media at a concentration in the range of about 100 nM to about 300 µM. In some cases the agonist concentration is in the range of about 1 µM to about 10 µM. After exposure to the agonist, the resulting agonist treated cells have an enhanced ability to bind to a cognate ligand." However, the inventor failed to recognize that media itself containing the small molecule could be used as a medical device (*e.g*., medium or cell carrier) alone or be part of cell treatment instrument for the ex vivo treatment of HSCs and other progenitor cells.

Consistent with this application, Vanderslice et al showed that the use of such agonists to improve the adhesion endothelial progenitor cells and HSCs for cardiac applications. However, the use of such agonists to enhance the homing and grafting of HSCs to bone marrow for treatment of hematologic cancers or genetic diseases has not been contemplated. Although very late antigen-4 (VLA-4) integrin and VCAM-1 interactions are known to mediated homing and grafting HSC to bone marrow stoma, Vanderslice et al failed to recognize that the use of such agonist for the engraftment of hematopoietic stem cells to reconstitute bone marrow compromised due to myeloablation therapy or inherently due to hematological cancer or genetic disease. HSCs express VLA-4 and such expression is known to mediate homing and grafting of HSC to bone marrow and medicate the reconstitution blood borne cells of myeloid and other lineages. In spite of the fact, Vanderslice et al showing that the adhesion of a cell line expressing VLA-4 to a VCAM-1 surface (as detailed below) is enhanced by small molecule agonist of very late antigen-4 (VLA-4) integrin, these investigators did not recognize that HSCs, which express VLA-4, could be treated *ex vivo* to improve bone marrow reconstitution and improve the outcomes of treatment modalities for a variety of hematologic cancers and genetic diseases.

In these studies, the TF-1 cells, a hematopoietic progenitor cell line, were incubated on surface containing VCAM-1 and exposed to high shear flow and the number of adherent cells were measured. VLA-4 agonists have been shown to significantly increase the rate and extent of adhesion of cells to this surface. In reviewing these data, the applicant discovered that as the compound (THI0019) stabilizes VLA-4 and other intergrins into a higher affinity state, and thereby increases the endogenous ligand binding potential, and upon ligand binding the compound is displaced, the compound could be removed from an ex vivo cell treatment media and cells suspended in a carrier that is essentially free of the compound could be suffused into a patient without loss of homing and granting.

While several methods and compositions have been proposed for homing and grafting of HSCcells, there is a continued need in the art for new methodologies for efficient, effective, safe and low cost enhancement of homing and grafting of hematopoietic stem cells to target tissues such as bone marrow.

### SUMMARY OF THE INVENTION

According to a first aspect, there is provided a composition for use in treating a patient having a cancer or a genetic disorder treatable using hematopoietic stem cells, according to claim 1. In certain embodiments, integrins targeted by these compounds include, but are not limited to, α4β1, α4β7, α5β1, αLβ2 and αVβ3. In various embodiments, ligands include, but are not limited to, VCAM-1, fibronectin, MAdCAM-1, ICAM-1, ICAM-2, and vitronectin.

According to a second aspect, there is provided a composition for use in treating a patient having a cancer or a genetic disorder treatable using hematopoietic stem cells, according to claim 2. In certain embodiments, integrins targeted by these compounds include, but are not limited to, α4β1, α4β7, α5β1, αLβ2 and αVβ3. In various embodiments, ligands include, but are not limited to, VCAM-1, fibronectin, MAdCAM-1, ICAM-1, ICAM-2, and vitronectin.

According to a third aspect, there is provided a cell carrier composition for use in treating a patient having a cancer or a genetic disorder treatable using hematopoietic stem cells, according to claim 3.In certain embodiments, integrins targeted by these compounds include, but are not limited to, α4β1, α4β7, α5β1, αLβ2 and αVβ3. In various embodiments, ligands include, but are not limited to, VCAM-1, fibronectin, MAdCAM-1, ICAM-1, ICAM-2, and vitronectin.

According to a fourth aspect, there is provided a composition for use in enhancing homing and grafting of hematopoietic stem cells (HSCs), wherein the composition is suffused into a target tissue of a patient, according to claim 8.. In certain embodiments, integrins targeted by these compounds include, but are not limited to, α4β1, α4β7, α5β1, αLβ2 and αVβ3. In various embodiments, ligands include, but are not limited to, VCAM-1, fibronectin, MAdCAM-1, ICAM-1, ICAM-2, and vitronectin.

The composition may include an additional amount of one or a plurality of chemical compounds capable of enhancing integrin-mediated binding of cells to their respective ligands.

### DEFINITIONS USED IN THE INVENTION

In addition to having their customary and usual meaning, the following definitions apply where the context permits in the specification and claims:

"Pharmaceutical composition" refers to a mixture of one or more chemicals, or pharmaceutically acceptable salts thereof, with a suitable carrier, for administration to a mammal as a medicine.

"Therapeutically effective amount" refers to that amount of the compound being administered that will relieve at least to some extent one or more of the symptoms of the disorder being treated. For example, an amount of the compound effective to prevent, alleviate or ameliorate symptoms of disease or prolong the survival of the subject being treated.

With respect to a disease or disorder, the term "treatment" refers to preventing, deterring the occurrence of the disease or disorder, arresting, regressing, or providing relief from symptoms or side effects of the disease or disorder and/or prolonging the survival of the subject being treated.

The term "alkyl" as used herein alone or in combination refers to C₁-C₁₂ straight or branched, substituted or unsubstituted saturated chain radicals derived from saturated hydrocarbons by the removal of one hydrogen atom. Representative examples of alkyl groups include methyl, ethyl, n-propyl, iso-propyl, n-butyl, sec-butyl, iso-butyl, and tert-butyl among others.

The term "alkenyl", alone or in combination, refers to a substituted or unsubstituted straight-chain or substituted or unsubstituted branched-chain alkenyl radical containing from 2 to 10 carbon atoms. Examples of such radicals include, but are not limited to, ethenyl, E- and Z-pentenyl, decenyl and the like.

The term "alkynyl", alone or in combination, refers to a substituted or unsubstituted straight or substituted or unsubstituted branched chain alkynyl radical containing from 2 to 10 carbon atoms. Examples of such radicals include, but are not limited to ethynyl, propynyl, propargyl, butynyl, hexynyl, decynyl and the like.

The term "lower" modifying "alkyl", "alkenyl", "alkynyl" or "alkoxy" refers to a C₁-C₆ unit for a particular functionality. For example lower alkyl means C₁-C₆ alkyl.

The term "cycloalkyl" as used herein alone or in combination refers to a substituted or unsubstituted aliphatic ring system having 3 to 10 carbon atoms and 1 to 3 rings, including, but not limited to cyclopropyl, cyclopentyl, cyclohexyl, norbornyl, and adamantyl among others. Cycloalkyl groups can be unsubstituted or substituted with one, two or three substituents independently selected from lower alkyl, haloalkyl, alkoxy, thioalkoxy, amino, alkylamino, dialkylamino, hydroxy, halo, mercapto, nitro, carboxaldehyde, carboxy, alkoxycarbonyl and carboxamide. This term is meant to encompass cycloalkenyl and cycloalkynyl groups. "Cycloalkyl" includes cis or trans forms. Furthermore, the substituents may either be in endo or exo positions in the bridged bicyclic systems.

The term "cycloalkenyl" as used herein alone or in combination refers to a cyclic carbocycle containing from 4 to 8 carbon atoms and one or more double bonds. Examples of such cycloalkenyl radicals include, but are not limited to, cyclopentenyl, cyclohexenyl, cyclopentadienyl and the like.

The term "cycloalkylalkyl" as used herein refers to a cycloalkyl group appended to a lower alkyl radical, including, but not limited to cyclohexyl methyl.

The term "halo" or "halogen" as used herein refers to I, Br, Cl or F.

The term "haloalkyl" as used herein refers to a lower alkyl radical, to which is appended at least one halogen substituent, for example chloromethyl, fluoroethyl, trifluoromethyl and pentafluoroethyl among others.

The term "alkoxy", alone or in combination, refers to an alkyl ether radical, wherein the term "alkyl" is as defined above. Examples of suitable alkyl ether radicals include, but are not limited to, methoxy, ethoxy, n-propoxy, iso-propoxy, n-butoxy, iso-butoxy, sec-butoxy, tert-butoxy and the like.

The term "alkenoxy", alone or in combination, refers to a radical of formula alkenyl-OB, provided that the radical is not an enol ether, wherein the term "alkenyl" is as defined above. Examples of suitable alkenoxy radicals include, but are not limited to, allyloxy, EB and Z-3-methyl-2-propenoxy and the like.

The term "alkynoxy", alone or in combination, refers to a radical of formula alkynyl-O B, provided that the radical is not an -ynol ether. Examples of suitable alkynoxy radicals include, but are not limited to, propargyloxy, 2-butynyloxy and the like.

The term "carboxyl" as used herein refers to BCO₂H.

The term "thioalkoxy", refers to a thioether radical of formula alkyl-SB, wherein "alkyl" is as defined above.

The term "carboxaldehyde" as used herein refers to BC(O)**R**, wherein **R** is hydrogen.

The term "carboxamide" as used herein refers to BC(O)N**R²**, wherein **R²** is hydrogen, alkyl or any other suitable substituent.

The term "alkoxyalkoxy" as used herein refers to **R^{b}**OB**R^{c}**OB, wherein **R^{b}** is lower alkyl as defined above and **R^{c}** is alkylene wherein alkylene is B(CH₂)_{n'}B, wherein n' is an integer from 1 to 6. Representative examples of alkoxyalkoxy groups include methoxymethoxy, ethoxymethoxy, and t-butoxymethoxy among others.

The term "alkylamino" as used herein refers to **R^{d}**NHB, wherein **R^{d}** is a lower alkyl group, for example, ethylamino, butylamino, among others.

The term "alkenylamino" alone or in combination, refers to a radical of formula alkenyl-NHB or (alkenyl)₂NB, wherein the term "alkenyl" is as defined above, provided that the radical is not an enamine. An example of such alkenylamino radicals is the allylamino radical.

The term "alkynylamino", alone or in combination, refers to a radical of formula alkynyl-NHB or (alkynyl)₂NB wherein the term "alkynyl" is as defined above, provided that the radical is not an amine. An example of such alkynylamino radicals is the propargyl amino radical.

The term "dialkylamino" as used herein refers to **R^{e}R^{f}**NB wherein **R^{e}** and **R^{f}** are independently selected from lower alkyl, for example diethylamino, and methyl propylamino, among others.

The term "amino" as used herein refers to H₂NB.

The term "alkoxycarbonyl" as used herein refers to an alkoxyl group as previously defined appended to the parent molecular moiety through a carbonyl group. Examples of alkoxycarbonyl include methoxycarbonyl, ethoxycarbonyl, and isopropoxycarbonyl among others.

The term "aryl" or "aromatic" as used herein alone or in combination refers to a substituted or unsubstituted carbocyclic aromatic group having about 6 to 12 carbon atoms such as phenyl, naphthyl, indenyl, indanyl, azulenyl, fluorenyl and anthracenyl; or a heterocyclic aromatic group selected from the group consisting of furyl, thienyl, pyridyl, pyrrolyl, oxazolyl, thiazolyl, imidazolyl, pyrazolyl, 2-pyrazolinyl, pyrazolidinyl, isoxazolyl, isothiazolyl, 1,2,3-oxadiazolyl, 1,2,3-triazolyl, 1,3,4-thiadiazolyl, pyridazinyl, pyrimidinyl, pyrazinyl, 1,3,5-triazinyl, 1,3,5-trithianyl, indolizinyl, indolyl, isoindolyl, 3H-indolyl, indolinyl, benzo[b]furanyl, 2,3-dihydrobenzofuranyl, benzo[b]thiophenyl, 1H-indazolyl, benzimidazolyl, benzthiazolyl, purinyl, 4H-quinolizinyl, isoquinolinyl, cinnolinyl, phthalazinyl, quinazolinyl, quinoxalinyl, 1,8-naphthridinyl, pteridinyl, carbazolyl, acridinyl, phenazinyl, phenothiazinyl, phenoxyazinyl, pyrazolo[1,5-c]triazinyl and the like. "Arylalkyl" and "alkylaryl" employ the term "alkyl" as defined above. Rings may be multiply substituted. Aromatic rings may be fused with other aromatic or non-aromatic rings to form multicyclic rings, and are also encompassed by the term "aromatic," as used herein.

The term "aralkyl", alone or in combination, refers to an aryl substituted alkyl radical, wherein the terms "alkyl" and "aryl" are as defined above. Examples of suitable aralkyl radicals include, but are not limited to, phenylmethyl, phenethyl, phenylhexyl, diphenylmethyl, pyridylmethyl, tetrazolyl methyl, furylmethyl, imidazolyl methyl, indolylmethyl, thienylpropyl and the like.

The term "aralkenyl", alone or in combination, refers to an aryl substituted alkenyl radical, wherein the terms "aryl" and "alkenyl" are as defined above.

The term "arylamino", alone or in combination, refers to a radical of formula aryl-N**R^{g}**-, wherein "aryl" is as defined above. **R^{g}** may be selected from the group consisting ofH, lower alkyl, aryl and aralkyl among others. Examples of arylamino radicals include, but are not limited to, phenylamino(anilido), naphthlamino, 2-, 3-, and 4-pyridylamino and the like.

The term "biaryl", alone or in combination, refers to a radical of formula aryl-aryl, wherein the term "aryl" is as defined above.

The term "thioaryl", alone or in combination, refers to a radical of formula aryl-S-, wherein the term "aryl" is as defined above. An example of a thioaryl radical is the thiophenyl radical.

The term "aroyl", alone or in combination, refers to a radical of formula aryl-COB, wherein the term "aryl" is as defined above. Examples of suitable aromatic acyl radicals include, but are not limited to, benzoyl, 4-halobenzoyl, 4-carboxybenzoyl, naphthoyl, pyridylcarbonyl and the like.

The term "heterocyclyl", alone or in combination, refers to a non-aromatic 3- to 10-membered ring containing at least one endocyclic N, O, or S atom. The heterocycle may be optionally aryl-fused. The heterocycle may also optionally be substituted with at least one substituent which is independently selected from the group consisting of hydrogen, halogen, hydroxyl, amino, nitro, trifluoromethyl, trifluoromethoxy, alkyl, aralkyl, alkenyl, alkynyl, aryl, cyano, carboxyl, alkoxycarbonyl, carboxyalkyl, oxo, arylsulfonyl and aralkylaminocarbonyl among others.

The term "alkylheterocyclyl" as used herein refers to an alkyl group as previously defined appended to the parent molecular moiety through a heterocyclyl group.

The term "heterocyclylalkyl" as used herein refers to a heterocyclyl group as previously defined appended to the parent molecular moiety through an alkyl group.

The term "aminal" as used herein refers to a hemi-acetal of the structure **R**CH(NH₂)(OH).

The terms "electron-withdrawing" or "electron-donating" refer to the ability of a substituent to withdraw or donate electrons relative to that of hydrogen if hydrogen occupied the same position in the molecule. These terms are well-understood by one skilled in the art and are discussed in ADVANCED ORGANIC CHEMISTRY by J. March, 1985, pp. 16-18. Electron withdrawing groups include halo, nitro, carboxyl, lower alkenyl, lower alkynyl, carboxaldehyde, carboxyamido, aryl, quaternary ammonium, trifluoromethyl, and aryl lower alkanoyl among others. Electron donating groups include such groups as hydroxy, lower alkyl, amino, lower alkylamino, di(lower alkyl)amino, aryloxy, mercapto, lower alkylthio, lower alkylmercapto, and disulfide among others. One skilled in the art will appreciate that the aforesaid substituents may have 53925652-1 electron donating or electron withdrawing properties under different chemical conditions. Moreover, the present invention contemplates any combination of substituents selected from the above-identified groups.

The most preferred electron donating or electron withdrawing substituents are halo, nitro, alkanoyl, carboxaldehyde, arylalkanoyl, aryloxy, carboxyl, carboxamide, cyano, sulfonyl, sulfoxide, heterocyclyl, guanidine, quaternary ammonium, lower alkenyl, lower alkynyl, sulfonium salts, hydroxy, lower alkoxy, lower alkyl, amino, lower alkylamino, di(lower alkyl)amino, amine lower alkyl mercapto, mercaptoalkyl, alkylthio and alkyldithio.

Use of the above terms is meant to encompass substituted and unsubstituted moieties. Substitution may be by one or more groups such as alcohols, ethers, esters, amides, sulfones, sulfides, hydroxyl, nitro, cyano, carboxy, amines, heteroatoms, lower alkyl, lower alkoxy, lower alkoxycarbonyl, alkoxyalkoxy, acyloxy, halogens, trifluoromethoxy, trifluoromethyl, alkyl, aralkyl, alkenyl, alkynyl, aryl, cyano, carboxy, carboalkoxy, carboxyalkyl, cycloalkyl, cycloalkylalkyl, heterocyclyl, alkylheterocyclyl, heterocyclylalkyl, oxo, arylsulfonyl and aralkylaminocarbonyl or any of the substituents of the preceding paragraphs or any of those substituents either attached directly or by suitable linkers. The linkers are typically short chains of 1-3 atoms containing any combination of-C-, -C(O)-, -NH-, -S-, -S(O)-, -O-, -C(O)O- or -S(O)O-. Rings may be substituted multiple times.

The term "mammals" includes humans and other animals.

The term "heteroatom" as used herein encompasses nitrogen, sulfur and oxygen.

The term "alpha" as used herein indicates the position immediately adjacent to the position described.

The term "inactive ingredient" as used herein indicated a harmless drug that is ordinarily used as an inactive ingredient, such as a coloring, emulsifier, excipient, flavoring, lubricant, preservative, or solvent, in the preparation of other drugs shall be exempt from section 502(f)(1) of the act (21 CFR 201.117).

The term "excipient" as used herein means any substance other than the active drug or product which has been appropriately evaluated for safety and is included in a drug delivery system to either aid the processing of the drug delivery system during its manufacture; protect, support, or enhance stability, bioavailability, or patient acceptability; assist in product identification; or enhance any other attribute of the overall safety and effectiveness of the drug delivery system during storage or use (40 CFR 63.1251).

The term "cell carrier" as used herein means any isotonic solution used as a medium to suspend cells.

The term "hematopoietic stem cell" as used herein means any cell that has the potential to self-renew, home and graft into bone marrow and/or differentiate in other blood cells such as cells of myeloid lineage, including without limitations neutrophils and platelets.

The term "intraossous infusion" as used herein means the route of administration of any agent by direct injection into bone marrow.

The term "minimally manipulated" as used herein means that the processing of cells does not alter the original relevant biological characteristics.

### ABBREVIATIONS USED IN THE INVENTION

The following abbreviations are used herein: Ac is acetyl, AcOH is acetic acid, 6-Ahx-OH is 6-aminohexanoic acid, Bn is benzyl, Boc is tert-butyloxycarbonyl, nBu is n-butyl, nBuLi is n-butyllithium, 1.6M in hexanes (unless other concentration noted), Cbz is benzyloxycarbonyl, CDI is N,N'-carbonyldiimidazole, COMU is (1-cyano-2-ethoxy-2-oxoethylidenaminooxy) dimethylamino-morpholino-carbenium hexafluorophosphate, Dab is 2,4-diaminobutyryl, DBU is 1,8-diazabicyclo[5.4.0]undec-7-ene, DCE is 1,2-dichloroethane, DCHA is dicyclohexylamine, DCM is dichloromethane (methlyene chloride),dioxane is 1,4-dioxane, DIPEA is N,N-diisopropylethylamine, DMED is N,N'-dimethylethylene diamine, DMF is N,N-dimethylformamide, DMSO is dimethylsulfoxide Et is ethyl, EtOH is ethanol, Fmoc is 9H-fluoren-9-ylmethyloxycarbonyl, Glu is glutamic acid, Gly is glycine, HBTU is 0-(Benzotriazol-1-yl)-N,N,N',N'-tetramethyluronium hexafluorophosphate, HMDS is hexamethyldisilazane, iPr is isopropyl, KHMDS is potassium bis(trimethylsilyl)amide, Lys is lysine, LHMDS is lithium bis(trimethylsilyl)amide, Me is methyl, MeOH is methanol, Nle is norleucine, NMM is 4-methylmorpholine, NSMC is N-succinimidyl-N-methylcarbamate, OAc is acetate, Orn is Ornithine, pTsOH is para-toluenesulfonic acid, Ph is phenyl, RT is room temperature, tBu is tert-butyl, TEA is triethylamine, Tfa is trifluoroacetyl, THF is tetrahydrofuran, Tol is toluene, Tyr is tyrosine, and Z is benzyloxycarbonyl.

### DETAILED DESCRIPTION OF THE INVENTION

The inventor has found that certain small molecule compounds or mixtures thereof which enhance integrin-mediated adhesion may be beneficial as therapeutic agents to enhance homing and grafting of hematopoietic stem cells (HSCs) to target tissues. Accordingly, a group of chemical compounds have been synthesized which enhance the integrin-mediated binding of cells to their respective ligands, integrins targeted by these compounds include, but are not limited to, α4β1, α4β7, α5β1, αLβ2 and αVβ3. Corresponding ligands include, but are not limited to, VCAM-1, fibronectin, MAdCAM-1, ICAM-1, ICAM-2 and vitronectin.

Agonist compounds, the ability of representative compounds to enhance binding of integrin-expressing cells, and therapeutic applications of agonist-treated cells are further described as follows.

In one embodiment of the present invention, a composition for media for HSCs is provided that comprises small molecule agonist of very late antigen-4 (VLA-4) integrin.

In another embodiment of the present invention, a composition for media for the ex vivo treatment of HSCs is provided that comprises small molecule agonist of very late antigen-4 (VLA-4) integrin.

In another embodiment of the present invention, a composition for media for the ex vivo treatment of HSCs is provided that comprises small molecule agonist of very late antigen-4 (VLA-4) integrin in sufficient amounts to enhance the adhesion of HSCs in bone marrow.

In another embodiment of the present invention, a composition comprising a HSC treated an *ex vivo* with a small molecule agonist of very late antigen-4 (VLA-4) integrin in sufficient amounts to enhance the adhesion of HSCs in bone marrow.

In another embodiment of the present invention, a composition comprising a HSC treated an *ex vivo* with a small molecule agonist of very late antigen-4 (VLA-4) integrin wherein the HSC is essential free of a small molecule agonist of very late antigen-4 (VLA-4), between 1 fM (1 femto molar or 1 H 10⁻¹⁵ M) and less than 100 nM (100 nano molar or 1 H 10⁻⁹ M), integrin prior to infusion of the HSC to enable adhesion of HSCs in bone marrow.

In another embodiment of the present invention, a composition for media to carry HSCs is provided that comprises small molecule agonist of very late antigen-4 (VLA-4) integrin in an electrolyte solution. Representative media or cell carrier may include without limitation; 1) Multiple Electrolytes Injection, Type 1, USP with nominal pH ranges of 5.5 to 8.0. Such media may be sterile, nonpyrogenic isotonic solution, 2) tissue culture media (eg. RPMI-1640 [RPMI]) without phenol red, 3) minimal media comprising of a saline solution (0.9% NaCl) containing 5% human serum albumin (Baxter or Talecris) and 8% Dextran 40 (Hospira) (LMD/HSA), 4) culture and expansion medium such as StemSpan-SFEM (Stem Cell Technologies), and 5) any isotonic solution comprising MnCl₂. Representative media may contain osmotic stabilizer of cells or cell membranes comprising, without limitation, protein derived from serum or plasma ( present in amounts from 0.5 wt.% to 50 wt.%).

In another embodiment of the present inventions, a composition for media for collecting HSCs, comprising of small molecule agonist of very late antigen-4 (VLA-4) integrin.

In another embodiment of the present inventions, a composition for media for transplantation of HSCs, comprising of small molecule agonist of very late antigen-4 (VLA-4) integrin.

In another embodiment of the present invention, a composition for transplantation comprising of small molecule agonist of very late antigen-4 (VLA-4) integrin, media, and therapeutically effective amount of HSCs, wherein the HSCs are derived from bone marrow, umbilical cord blood, peripheral blood, or wherein the HSCs consist essentially of CD34+ HSCs.

Also disclosed herein is a method for transplantation by administering a composition comprising of small molecule agonist of very late antigen-4 (VLA-4) integrin, media, and therapeutically effective amount of HSCs by intravenous, or intra-arterial injection.

Also disclosed herein is a method for transplantation by administering a composition comprising of small molecule agonist of very late antigen-4 (VLA-4) integrin, media, and therapeutically effective amount of HSCs by intraosseous infusion, wherein the site of access to the body is without limitation posterior iliac crest, tibia, or sternum.

Also disclosed herein is a method for transplantation by administering a composition comprising of small molecule agonist of very late antigen-4 (VLA-4) integrin, media, and therapeutically effective amount of HSCs by intravenous, or intra-arterial injection, wherein the HSC is pre-treated with small molecule agonist of very late antigen-4 (VLA-4) integrin, and prior to infusion into an animal the HSC is essential free of the small molecule agonist of very late antigen-4 (VLA-4) integrin.

Also disclosed herein is a method for transplantation by administering a composition comprising of small molecule agonist of very late antigen-4 (VLA-4) integrin, media, and therapeutically effective amount of HSCs by intraosseous infusion, wherein the site of access to the body is without limitation posterior iliac crest, tibia, or sternum, wherein the HSC is pre-treated with small molecule agonist of very late antigen-4 (VLA-4) integrin, and prior to infusion into an animal the HSC is essential free of the small molecule agonist of very late antigen-4 (VLA-4) integrin.

In another embodiment of the present invention, a composition comprising an infusion bag, small molecule agonist of very late antigen-4 (VLA-4) integrin, media to carry HSCs is provided that comprises small molecule agonist of very late antigen-4 (VLA-4) integrin in an electrolyte solution. Representative media may include without limitation; 1) Multiple Electrolytes Injection, Type 1, USP with nominal pH ranges of 5.5 to 8.0. Such media may be sterile, nonpyrogenic isotonic solution, 2) tissue culture media (eg. RPMI-1640 [RPMI]) without phenol red, 3) minimal media comprising of a saline solution (0.9% NaCl) containing 5% human serum albumin (Baxter or Talecris) and 8% Dextran 40 (Hospira) (LMD/HSA), 4) culture and expansion medium such as StemSpan-SFEM (Stem Cell Technologies) and 5) any isotonic solution comprising MnCl₂.

Also disclosed herein is a medical device comprising an infusion bag containing a small molecule agonist of very late antigen-4 (VLA-4) integrin, wherein the small molecule compound facilitating the interaction of very late antigen-4 (VLA-4) integrin to an endogenous ligand is an inactive ingredient and/or excipient.

Compounds of this invention have the ability to enhance binding of integrin-expressing cells, and therapeutic applications of agonist-treated cells are further described as follows.

### General Compositions and Methods

Embodiments of this invention relate to compositions including hematopoietic stem cells (HSCs), and an effective amount one or a plurality of chemical compounds capable of enhancing integrin-mediated binding of cells to their respective ligands, where the effective amount between about 1 fM and about 300 µM and where the composition is used to treat a patient having a cancer or a genetic disorder treatable using hematopoietic stem cells. In certain embodiment, the cancer or genetic disorder is selected from the group consisting of acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, and Diamond Blackfan syndrome. In other embodiments, the integrins targeted by these compounds are selected from the groups consisting of α4β1, α4β7, α5β1, αLβ2, αVβ3, and mixtures or combinations thereof. In other embodiments, the ligands are selected from the group consisting of VCAM-1, fibronectin, MAdCAM-1, ICAM-1, ICAM-2, vitronectin, and mixtures or combinations thereof. Tthe chemical compounds are given by the general formula (I):

**R¹ - M¹ -N(R²)-M² - M³ - M⁴ - M⁵ - M⁶ - R³** (I)

where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein **q** is an integer from 1 to 6, and
**R³** is CON**R¹³R¹⁴**, where **R¹³** and **R¹⁴** are independently aralkyl

In other embodiments, the effective amount in carrier is greater than 1 fM and less than 100 nM. In other embodiments, the effective amount in carrier is greater than 1 fM and less than 50 nM. In other embodiments, the effective amount in carrier is greater than 1 fM and less than 25 nM.

Embodiments of this invention relates to compositions including treated hematopoietic stem cells (tHSCs) comprising hematopoietic stem cells (HSCs) treated with one or a plurality of chemical compounds capable of enhancing integrin-mediated binding of cells to their respective ligands, where the composition is used to treat a patient having a cancer or a genetic disorder treatable using hematopoietic stem cells. In certain embodiments, the cancer or genetic disorder is selected from the group consisting of acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, and Diamond Blackfan syndrome. In other embodiments, the integrins targeted by these compounds are selected from the groups consisting of α4β1, α4β7, α5β1, αLβ2, αVβ3, and mixtures or combinations thereof. In other embodiments, the ligands are selected from the groups consisting of VCAM-1, fibronectin, MAdCAM-1, ICAM-1, ICAM-2, vitronectin, and mixtures or combinations thereof. The chemical compounds are given by the general formula (I):

**R¹ - M¹ -N(R²)-M² - M³ - M⁴ - M⁵ - M⁶ - R³** (I)

where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein **q** is an integer from 1 to 6, and
**R³** is CON**R¹³R¹⁴**, where **R¹³** and **R¹⁴** are independently aralkyl

In other embodiments, the effective amount in carrier is greater than 1 fM and less than 100 nM. In other embodiments, the effective amount in carrier is greater than 1 fM and less than 50 nM. In other embodiments, the effective amount in carrier is greater than 1 fM and less than 25 nM.

Embodiments of this invention relates to methods for enhancing homing and grafting of hematopoietic stem cells (HSCs) including suffusing a composition into a target tissue of a patient, where the composition comprises hematopoietic stem cells (HSCs) and an effective amount of one or a plurality of chemical compounds capable of enhancing integrin-mediated binding of cells to their respective ligands, where the chemical compounds are present at an effective amount between about 1 fM and about 300 µM, and/or treated hematopoietic stem cells (tHSCs) comprising hematopoietic stem cells (HSCs) treated with one or a plurality of chemical compounds capable of enhancing integrin-mediated binding of cells to their respective ligands, where the target tissue is bone marrow and where the patient has a cancer or a genetic disorder treatable using hematopoietic stem cells. In certain embodiments, further include washing the treated hematopoietic stem cells until a concentration of the chemicals compounds is between about 1 fM and about 300 µM. In other embodiments, the cancer or genetic disorder is selected from the group consisting of acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, and Diamond Blackfan syndrome. In other embodiments, the integrins targeted by these compounds are selected from the groups consisting of α4β1, α4β7, α5β1, αLβ2, αVβ3, mixtures or combinations thereof. In other embodiments, the ligands are selected from the groups consisting of VCAM-1, fibronectin, MAdCAM-1, ICAM-1, ICAM-2, vitronectin, and mixtures or combinations thereof The chemical compound are given by the **general formula (I)**:

**R¹ - M¹ -N(R²)-M² - M³ - M⁴ - M⁵ - M⁶ - R³** (I)

where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein **q** is an integer from 1 to 6, and
**R³** is CON**R¹³R¹⁴**, where **R¹³** and **R¹⁴** are independently aralkyl

In other embodiments, the effective amount in carrier is greater than 1 fM and less than 100 nM. In other embodiments, the effective amount in carrier is greater than 1 fM and less than 50 nM. In other embodiments, the effective amount in carrier is greater than 1 fM and less than 25 nM.

Embodiments of this invention relate to cell carrier compositions including hematopoietic stem cells (HSCs), and an effective amount of one or a plurality of chemical compounds capable of enhancing integrin-mediated binding of cells to their respective ligands, and/or treated hematopoietic stem cells (tHSCs) comprising hematopoietic stem cells (HSCs) treated with one or a plurality of chemical compounds capable of enhancing integrin-mediated binding of cells to their respective ligands, where the effective amount between about 1 fM and about 300 µM and the composition is used to treat a patient having a cancer or a genetic disorder treatable using hematopoietic stem cells. In certain embodiments, the cancer or genetic disorder is selected from the group consisting of acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, or Diamond Blackfan syndrome. In other embodiments, the integrins targeted by these are selected from the groups consisting of α4β1, α4β7, α5β1, αLβ2, αVβ3, and mixtures or combinations thereof. In other embodiments, the ligands are selected from the groups consisting of VCAM-1, fibronectin, MAdCAM-1, ICAM-1, ICAM-2, vitronectin, and mixtures or combinations thereof. The chemical compound are given by the general formula (I):

**R¹ - M¹ -N(R²)-M² - M³ - M⁴ - M⁵ - M⁶ - R³** (I)

where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein **q** is an integer from 1 to 6, and
**R³** is CON**R¹³R¹⁴**, where **R¹³** and **R¹⁴** are independently aralkyl

. In other embodiments, the effective amount in carrier is greater than 1 fM and less than 100 nM. In other embodiments, the effective amount in carrier is greater than 1 fM and less than 50 nM. In other embodiments, the effective amount in carrier is greater than 1 fM and less than 25 nM.

### Agonist Pre-treated Cells

One or more integrin-expressing cells are first treated (pre-treated) with an agonist compound having the general formula I, as described herein, to form agonist-bound integrin molecules on the cell's surface. The integrin-expressing cells may be embryonic stem cells, adult stem/progenitor cells, or induced pluripotent stem cells, for example. In some cases, the cells express one or more of the integrins α4β1, α5β1, α4β7, αvβ3 and αLβ2. The treatment of the cells generally includes contacting the integrin-expressing cells in vitro with the agonist. In most applications the agonist compound in present in the treatment media at a concentration in the range of about 100 nM to about 30 µM. In some cases the agonist concentration is in the range of about 1 µM to about 10 µM. In other cases, the agonist may be removed by washing with an isotonic solution such that the media, cell carrier, or cell suspension is essentially free of the agonist. After exposure to the agonist, the resulting agonist-treated cells have an enhanced ability to bind to a cognate ligand. The integrin is expressed on the surface of the cells, and may be either naturally occurring or transgenically expressed by a cell that has been transformed to express an exogenous integrin gene. The protein or other cognate ligand to which the integrin binds is expressed either on a cell surface or is part of the extracellular matrix.

### Enhanced Binding of Pre-Treated Cells to Integrin-Binding Ligands

The agonist, as described herein, dissolved in a pharmaceutically acceptable diluent, is added to cell culture media or cell suspension and mixed. The resulting agonist-treated cells are introduced to an integrin-binding ligand or binding site, whereupon the treated cells bind, attach or adhere to the cognate ligands in solution, or on a surface or target tissue. In some cases an integrin binding protein is vascular cell adhesion molecule-1 (VCAM 1), fibronectin, mucosal addressin cellular adhesion molecule-1 (MAdCAM-1), intercellular adhesion molecule-1 (ICAM-1), intercellular adhesion molecule-2 (ICAM-2) or vitronectin. As a result of the agonist treatment, the binding of the agonist-treated cells to the ligand is enhanced or increased compared to binding of integrin-expressing cells not treated with the agonist. In some cases, at least 3 fold more agonist-treated cells are bound to a ligand-coated surface than untreated integrin-expressing cells. In some cases, up to 3 fold more agonist-treated cells than untreated cells are bound to an integrin binding protein.

### Enhanced Retention of Pre-Treated Cells to Tissues Expressing Integrin-Binding Ligands

Regardless of the cell type, mechanism of action, or how they are delivered, for many applications it is critical that the cells home to, and are retained in bone marrow. Low levels of cell retention observed in animal models and clinical trials are considered one of the major impediments to the progress of HSC-based therapies. Even when cells are injected locally, less than 10% of injected cells are typically retained after one hour and this number decreases over time in conventional cell-based therapies. The retention rates are even lower when delivered systemically. By comparison, many embodiments of the presently disclosed methods increase the rate of retention of exogenously delivered cells and will potentially greatly further efforts in regenerative medicine.

A method of enhancing retention of exogenously-introduced cells at an *in vivo* target site in a mammal generally includes (a) treating integrin-expressing cells *in vitro* with an agonist of integrin, wherein the agonist is a compound having the general formula I, as described herein; (b) optionally removing the agonist such that integrin expressing cell is essentially free of the agonist; (c) introducing the agonist-treated cells to an in vivo target site in the mammal; and (d) causing a greater number of said introduced agonist-treated cells to remain at said target site relative to the number of cells retained if integrin-expressing cells not treated with said agonist were introduced to said target site. The target site includes an integrin binding protein such as vascular cell adhesion molecule-1 (VCAM 1), fibronectin, mucosal addressin cellular adhesion molecule-1 (MAdCAM-1), inter-cellular adhesion molecule-1 (ICAM-1), inter-cellular adhesion molecule-2 (ICAM-2) or vitronectin, for example.

A method of enhancing retention of exogenously-introduced cells at an *in vivo* target site in a patient that has acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, or Diamond Blackfan syndrome.

### Media Compositions

The compounds used in the *ex vivo* media production described herein may be used in the form of pharmaceutically acceptable salts derived from inorganic or organic acids. The phrase "pharmaceutically acceptable salt" means those salts which are, within the scope of sound medical judgment, suitable for use in contact with the tissues of humans and lower animals without undue toxicity, irritation, allergic response and the like and are commensurate with a reasonable benefit/risk ratio. Pharmaceutically acceptable salts are well-known in the art. For example, S. M. Berge et al. describe pharmaceutically acceptable salts in detail in J. Pharmaceutical Sciences, 1977, 66: 1 et seq. The salts may be prepared in situ during the final isolation and purification of the compounds or separately by reacting a free base function with a suitable organic acid. Representative acid addition salts include, but are not limited to acetate, adipate, alginate, citrate, aspartate, benzoate, benzenesulfonate, bisulfate, butyrate, camphorate, camphor sulfonate, digluconate, glycerophosphate, hemisulfate, heptanoate, hexanoate, fumarate, hydrochloride, hydrobromide, hydroiodide, 2-hydroxyethansulfonate (isothionate), lactate, maleate, methane sulfonate, nicotinate, 2-naphthalene sulfonate, oxalate, palmitoate, pectinate, persulfate, 3-phenylpropionate, picrate, pivalate, propionate, succinate, tartrate, thiocyanate, phosphate, glutamate, bicarbonate, p-toluenesulfonate and undecanoate. Also, the basic nitrogen-containing groups can be quaternized with such agents as lower alkyl halides such as methyl, ethyl, propyl, and butyl chlorides, bromides and iodides; dialkyl sulfates like dimethyl, diethyl, dibutyl and diamyl sulfates; long chain halides such as decyl, lauryl, myristyl and stearyl chlorides, bromides and iodides; arylalkyl halides like benzyl and phenethyl bromides and others. Water or oil-soluble or dispersible products are thereby obtained. Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid and citric acid.

In some embodiments, basic addition salts are prepared in situ during the final isolation and purification of a disclosed compound by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium and aluminum salts and the like and nontoxic quaternary ammonia and amine cations including ammonium, tetramethylammonium, tetraethylammonium, methylammonium, dimethylammonium, trimethylammonium, triethylammonium, diethylammonium, and ethylammonium among others. Other representative organic amines useful for the formation of base addition salts include ethylenediamine, ethanolamine, diethanolamine, piperidine, piperazine and the like.

Actual concentration of ingredients in the media compositions may be varied so as to obtain an amount of the compound(s) which is effective to achieve the desired therapeutic response mediated by the cell therapeutic treated with media for a particular patient, compositions and mode of administration. The selected concentration level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

When used for the production of cells for various therapeutic treatments, a therapeutically effective amount of one or more of the disclosed compounds be employed in pure form or, where such forms exist, in pharmaceutically acceptable salt, ester or pro-drug form. In some cases, the compound is administered as a pharmaceutical composition containing the compound of interest in combination with one or more other pharmaceutically acceptable inactive ingredients or excipients. The phrase "therapeutically effective amount" of a disclosed compound means a sufficient amount of the compound to generate a cell therapeutic composition to treat disorders, at a reasonable benefit/risk ratio applicable to any medical treatment. The specific therapeutically effective concentration of the compound and cell dose for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start cell doses of the compound at levels lower than required to achieve the desired therapeutic effect and to gradually increase the dosage until the desired effect is achieved.

The concentration of the disclosed compounds in the *ex vivo* treatment media alone or in combination with therapeutic cells in a suitable media to infuse a human or lower animal may be between 1 fM (1 femto molar or 1 H 10⁻¹⁵ M) and less than 10 µM. If desired, the effective concentration can be divided into multiple doses for purposes of administration; consequently, single dose compositions may contain such amounts or submultiples thereof to make up the daily dose of cell or compound.

The total daily concentrations of the disclosed compounds administered in combination with therapeutic cells in a suitable media to a human or lower animal may between 1 fM (1 femto molar or 1 H 10⁻¹⁵ M) and less than 10 µM. If desired, the effective daily dose can be divided into multiple doses for purposes of administration; consequently, single dose compositions may contain such amounts or submultiples thereof to make up the daily dose of cell or exposure of the compound.

Compositions suitable for parenteral injection may comprise physiologically acceptable, sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propyleneglycol, polyethyleneglycol, glycerol, and the like), vegetable oils (such as olive oil), injectable organic esters such as ethyl oleate, and suitable mixtures thereof. These compositions can also contain adjuvants such as preserving, wetting, emulsifying, and dispensing agents. Prevention of the action of microorganisms can be ensured by various antibacterial and antifungal agents, for example, parabens, chlorobutanol, phenol, sorbic acid, and the like. It may also be desirable to include isotonic agents, for example sugars, sodium chloride and the like. Prolonged absorption of the injectable pharmaceutical form can be brought about by the use of agents delaying absorption, for example, aluminum monostearate and gelatin.

Suspensions, in addition to the active compounds, may contain suspending agents, as for example, ethoxylated isostearyl alcohols, polyoxyethylene sorbitol and sorbitan esters, microcrystalline cellulose, aluminum metahydroxide, bentonite, agar-agar and tragacanth, or mixtures of these substances, and the like. Proper fluidity can be maintained, for example, by the use of coating materials such as lecithin, by the maintenance of the required particle size in the case of dispersions and by the use of surfactants.

In some cases, for compounds with minimal solubility, solubility enhancers without limitation include surfactants such as zwitterionic phospholipids, non-esterified fatty acids, mono-, di- or triglycerides alone or in combinations secondary surfactants may be used.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

In some embodiments, a chemical compound is provided having the general **formula (I)**

**R¹ - M¹ -N(R²)-M² - M³ - M⁴ - M⁵ - M⁶ - R³** (I)

where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein **q** is an integer from 1 to 6, and
**R³** is CON**R¹³R¹⁴**, where **R¹³** and **R¹⁴** are independently aralkyl

In some embodiments, the compound is selected from the group consisting of methyl (6S,10S)-10-(1,3-benzodioxol-5-yl)-6-butyl-3,8-dioxo-1-(2-thienyl)-2-(2-thienylmethyl)-4-oxa-2,7,9-triazadodecan-12-oate; methyl(6S,10R)-10-(1,3-benzodioxol-5-yl)-6-butyl-3,8-dioxo-1-(2-thienyl)-2-(2-thienylmethyl)-4-oxa-2,7,9-triazadodecan-12-oate; methyl(6S,10S)-10-(1,3-benzodioxol-5-yl)-6-butyl-7-methyl-3,8-dioxo-1-(2-thienyl)-2-(2-thienylmethy 1)-4-oxa-2,7,9-triazadodecan-12-oate; methyl(6S,10S)-10-(1,3-benzodioxol-5-yl)-6-butyl-9-methyl-3,8-dioxo-1-(2-thienyl)-2-(2-thienyl)-2-(2-thienylmethyl)-4-oxa-2,7,9-triazadodecan-12-oate; ethyl(6S,10R)-10-(1,3-benzodioxol-5-yl)-6-butyl-7-methyl-3,8-dioxo-1-(-2-thienyl)-2-(2-thienylmethyl)-4-oxa-2,7,9-triazadodecan-12-oate; methyl(10S)-10-(1,3-benzodioxol-5-yl)-3,8-dioxo-1-(2-thienyl)-2-(2-thienylmethyl)-4-oxa-2,7,9-triazadodecan-12-oate; methyl 3,8-dioxo-1-(2-thienyl)-2-(2-thienylmethyl)-4-oxa-2,7,9-triazadodecan-12-oate; methyl(6S,10S)-10-(1,3-benzodioxol-5-yl)-6-butyl-2-methyl-3,8-dioxo-1-(2-thienyl)-4-oxa-2,7,9-triazadodecan-12-oate; methyl(6S)-6-butyl-3,8-dioxo-1-(2-thienyl)-2-(2-thienylmethyl)-4-oxa-2,7,-9-triazadodecan-12-oate; (2S)-2-{[(1,3-benzodioxol-5-ylmethyl)carbamoyl]amino}hexyl bis(2-thienylmethyl)carbamate; methyl(6S,10S)-6-butyl-3,8-dioxo-10-phenyl-1-(2-thienyl)-2-(2-thienylmethyl)-4-oxa-2,7,9-triazadode can-12-oate; (2S)-2-({[(1S)-1-(1,3-benzodioxol-5-yl)- 3-hydroxypropyl]carbamoyl}amino)hexyl-bis(2-thienylmethyl)carbamate; (2S)-2-[(benzylcarbamoyl)amino]hexyl-bis(2-thienylmethyl)carbamate; (2S)-2-[(morpholin-4-ylcarbonyl)amino]hexyl-bis(2-thienylmethyl)carbamate; (2S)-2-{[(3-methoxypropyl)carbamoyl]amino}hexyl-bis(2-thienylmethyl)carbamate; (2S)-2-{[(2-methoxyethyl)carbamoyl]amino}hexyl-bis(2-thienylmethyl)carbamate; tert-butyl[(2S)-1-{[bis(2-thienylmethyl)carbamoyl]oxy}hexan-2-yl]carbamate; (2S)-2-[(tert-butylcarbamoyl)amino]hexyl-bis(2-thienylmethyl)carbamate; (2S)-2-[(isopropylcarbamoyl)amino]hexyl-bis(2-thienylmethyl)carbamate; (2S)-2-[(methylcarbamoyl)amino]hexyl-bis(2-thienylmethyl)carbamate; tert-butyl[(2R)-1-{[bis(2-thienylmethyl)carbamoyl]oxy}hexan-2-yl]carbamate; benzyl{(5S)-6-{[bis(2-thienylmethyl)carbamoyl]oxy}-5-[(tert-butoxycarbonyl)amino]hexyl}carbamate; methyl(9S,13S)-13-(1,3-benzodioxol-5-yl)-9-({[bis(2-thienylmethyl)carbamoyl]oxy}methyl)-3,11-dioxo-1-phenyl-2-oxa-4,10,12-triazapentadecan-15-oate; (2S)-2-acetamidohexyl bis(2-thienylmethyl)carbamate; methyl(3R)-3-(1,3-benzodioxol-5-yl)-3-{[(2S)-2-{[bis(2-thienylmethyl)carbamoyl]amino}hexanoyl]amino}propanoate; methyl(3R)-3-(l,3-benzodioxol-5-yl)-3-{[(2R)-2-{[bis(2-thienylmethyl)carbamoyl]amino} hexanoyl]amino}propanoate; methyl(3S)-3-(1,3-benzodioxol-5-yl)-3-{[(2R)-2-{[bis(2-thienylmethyl)carbamoyl]amino}hexanoyl]amino}propanoate; methyl(6R,10S)-10-(1,3-benzodioxol-5-yl)-6-butyl-3,8-dioxo-1-(2-thienyl)-2-(2-thienylmethyl)-4-oxa-2,7,9-triazadodecan-12-oate; methyl(6R,10R)-10-(1,3-benzodioxol-5-yl)-6-butyl-3,8-dioxo-1-(2-thienyl)-2-(2-thienylmethyl)-4-oxa-2,7,9-triazadodecan-12-oate; methyl(2S)-2-{[bis(2-thienylmethyl)-carbamoyl]amino}hexanoate; methyl(2R)-2-{[bis(2-thienylmethyl)carbamoyl] amino }hexanoate; 3-[(2S)-1-hydroxyhexan-2-yl]-1,1-bis(2-thienylmethyl)urea; 3-[(2R)-1-hydroxyhexan-2-yl]-1,1-bis(2-thienylmethyl)urea; methyl(2S)-6-{[(benzyloxy)carbonyl]amino}-2-{[bis(2-thienylmethyl)carbamo-yl]amino}hexanoate; methyl{[bis(2-thienylmethyl)carbamoyl](methyl)amino}acetate; methyl{[bis(2-thienylmethyl)carbamoyl]amino}acetate; methyl {[bis(2-thienylmethyl)carbamoyl](butyl)amino}acetate; 3-(3-hydroxypropyl)-1,1-bis(2-thienylmethyl)urea; methyl(2R)-{[bis(2-thienylmethyl)carbamoyl]amino}(phenyl)acetate; tert-butyl{[bis(2-thienylmethyl)carbamoyl]amino}acetate; tert-butyl{[bis(2-thienylmethyl)carbamoyl](butyl)amino}acetate; benzyl{(5S)-6-{[bis(4-methoxybenzyl)carbamoyl]oxy}-5-[(tert-butoxycarbonyl)amino]hexyl}carbamat e; tert-butyl[(2S)-1-{[bis(4-methoxybenzyl)carbamoyl]oxy}hexan-2-yl]carbamate; methyl(6S,10S)-10-(1,3-benzodioxol-5-yl)-6-butyl-2-(4-methoxybenzyl)-1-(4-methoxyphenyl)-3,8-dioxo-4-oxa-2,7,9-triazadodecan-12-oate; (2S)-2-({[(1S)-1-(1,3-benzodioxol-5-yl)-3-hydroxypropyl]carbamoyl}amino)hexyl bis(4-methoxybenzyl)carbamate; (2S)-2-[(tert-butoxycarbonyl)amino]hexyl dibenzylcarbamate; methyl(6S,10S)-10-(1,3-benzodioxol-5-yl)-2-benzyl-6-butyl-3,8-dioxo-1-phenyl-4-oxa-2,7,9-triazadod ecan-12-oate; tert-butyl[(2S)-1{[bis(4-methylbenzyl)carbamoyl]oxy}hexan-2-yl]carbamate; methyl(6S,10S)-10-(1,3-benzodioxol-5-yl)-6-butyl-2-(4-methylbenzyl)-1-(4-methylphenyl)-3,8-dioxo-4-oxa-2,7,9-triazadodecan-12-oate; tert-butyl[(2S)-1-{[bis(4-chlorobenzyl) carbamoyl]oxy}hexan-2-yl]carbamate; methyl(6S,10S)-10-(1,3-benzodioxol-5-yl) -6-butyl-2-(4-chlorobenzyl)-1-(4--chlorophenyl)-3,8-dioxo-4-oxa-2,7,9-triazadodecan-12-oate; (2S)-2-[(tert-butoxycarbonyl)amino]hexyl (4-bromobenzyl)(2-thienylmethyl)carbamate; methyl(6S,10S)-10-(1,3-benzodioxol-5-yl)-2-(4-bromobenzyl)-6-but-yl-3,8-dioxo-1-(2-thienyl)-4-oxa-2,7,9-triazadodecan-12-oate; methyl(6S,10S)-2-(4-azidoobenzyl)-10-(1,3-benzodioxol-5-yl)-6-butyl-3,8-dioxo-1-(2-thienyl)-4-oxa-2,7,9-triazadodecan-12-oate; (2S)-2-[(tert-butoxycarbonyl)amino]hexylphenyl(2-thienylmethyl)carbamate; methyl(6S,100S)-10-(1,3-benzodioxol-5-yl)-6-butyl-3,8-dioxo-2-phenyl-1-(2 -thienyl)-4-oxa-2,7,9-triazadodecan-12-oate; tert-butyl[(2S)-1-{[bis(3-thienylmethyl) carbamoyl]oxy}hexan-2-yl]carbamate; methyl(6S,10S)-10-(1,3-benzodioxol-5-yl) -6-butyl-3,8-dioxo-1-(3-thienyl)-2-(3-thienylmethyl)-4-oxa-2,7,9-triazadodecan-12-oate; benzyl[(5S)-5-[(tert-butoxycarbonyl)amino]-6-{[butyl(2-thienylmethyl)carbamoyl]oxy}hexyl]carbamate; (2S)-2-[(tert-butoxycarbonyl)amino]hexyl butyl(2-thienylmethyl)carbamate; methyl(3S,7S)-3-(1,3-benzodioxol-5-yl)-7-butyl-5,10-dioxo-[1-(2-thienylmethyl)-9-oxa-4,6,11-triazapentadecan-1-oate; benzyl[(5S)-5-[(tert-butoxycarbonyl)amino]-6-{[(2-methoxyethyl)(2-thienylmethyl)carbamoyl]oxy}hexyl]carbamate; (2S)-2-[(tert-butoxycarbonyl) amino]hexyl(2-methoxyethyl)(2-thienylmethyl)carbamate; methyl(9S,13S)-13-(1,3-benzodioxol-5-yl)-9-butyl-6,11-dioxo-5-(-2-thienylmethyl)-2,7-dioxa-5,10,12-triazapentadecan-15-oate; (2S)-2-[({3-[(methylsulfonyl) amino]benzyl}carbamoyl)amino]hexyl(2-methoxyethyl)(2-thienylmethyl)carbamate; (2S)-2-{[(4-bromobenzyl)carbamoyl]amino}hexyl-bis(2-thienylmethyl)carbamate; (2S)-2-{[(4-azidobenzyl)carbamoyl]amino}hexyl-bis(2-thienylmethyl)carbamate; tert-butyl[(2S)-1-{[bis(2-thienylmethyl)carbamoyl]thio}hexan-2-yl]carbamate; methyl(6S,10S)-10-(1,3-benzodioxol-5-yl)-6-butyl-3,8-dioxo-1-(2-thienyl)-2-(2-thienylmethyl)-4-thia-2,7,9-triazadodecan-12-oate; and mixtures or combinations thereof.

In some embodiments, a compound is selected from the group consisting of (2R)-2-({[(1S)-1-(1,3-benzodioxol-5-yl)-3-hydroxypropyl]carbamoyl}amino)-N,N-bis(2-thienylmethyl )hexanamide; methyl(3S)-3-(1,3-benzodioxol-5-yl)-3-[({3-[bis(2-thienylmethyl)amino]-3-oxopropyl}carbamoyl)amino]propanoate; (2S)-2-[(tert-butylcarbamoyl)amino]-N,N-bis(2-thienylmethyl)hexanamide; tert-butyl{(2S)-1-[bis(2-thienylmethyl)amino]-1-oxohexan-2-yl}carbamate; benzyl{(5S)-6-[bis(2-thienylmethyl)amino]-5-[(tert-butoxycarbonyl)(methyl)amino]-6-oxohexyl}carbamate; benzyl{(5S)-6-[bis(2-thienylmethyl)amino]-5-[(tert-butoxycarbonyl)amino]-6-oxohexyl}carbamate; benzyl{(5R)-6-[bis(2-thienylmethyl)amino]-5-[(tert-butoxycarbonyl)amino]-6-oxohexyl}carbamate; tert-butyl{(2R)-1-[bis(2-thienylmethyl)amino]-1-oxohexan-2-yl}carbamate; (2S)-2-acetamido-N,N-bis(2-thienylmethyl)hexanamide; benzyl{(5S)-5-acetamido-6-[bis(2-thienylmethyl)amino]-6-oxohexyl}carbamate; (2R)-2-acetamido-N,N-bis(2-thienylmethyl)hexanamide; benzyl{(5S)-5-(benzoylamino)-6-[bis(2-thienylmethyl)amino]-6-oxohexyl}carbamate; (2S)-2-[(phenylsulfonyl)amino]-N,N-bis(2-thienylmethyl)hexanamide; (2S)-2-[methyl(phenylsulfonyl)amino]-N,N-bis(2-thienylmethyl)hexanamide; 2-[(phenylsulfonyl)amino]-N,N-bis(2-thienylmethyl)acetamide; 2-[methyl(phenylsulfonyl)amino]-N,N-bis(2-thienylmethyl)acetamide; (2S)-2-[(methylsulfonyl)amino]-N,N-bis(2-thienylmethyl)hexanamide; (2S)-2-({[3-(4-methoxyphenoxy)propyl]sulfonyl}amino)-N,N-bis(2-thienylmethyl)hexanamide; benzyl{(5S)-6-[bis(2-thienylmethyl)amino]-6-oxo-5-[(2-thienylsulfonyl)amino]hexyl}carbamate; benzyl{(5S)-5-[(tert-butoxycarbonyl)amino]-6-[(3-methoxybenzyl)(2-thienyl-methyl)amino]-6-oxohex yl}carbamate; benzyl{(5S)-6-[bis(3-methoxybenzyl)amino]-5-[(tert-butoxycarbonyl)amino]-6-oxohexyl}carbamate; benzyl{(5R)-5-[(tert-butoxycarbonyl)amino]- 6-[(3-methoxybenzyl) (2-thienyl-methyl)amino]-6-oxohexyl}carbamate; benzyl{(5R)-6-[bis(3-methoxybenzyl)amino]-5-[(tert-butoxycarbonyl)amino]-6-oxohexyl}carbamate; benzyl[(5S)-5-[(tert-butoxycarbonyl)amino] -6-oxo-6-{[2-(2-thienyl)ethyl] (-2-thienylmethyl)amino }hexyl]carbamate; benzyl[(5R)-5-[(tert-butoxycarbonyl)amino]-6-oxo-6-{[2-(2-thienyl)ethyl](-2-thienylmethyl)amino} hexyl]carbamate; benzyl[(5S)- 5-[(tert-butoxycarbonyl)amino]-6-(dibenzylamino) -6-oxohexyl]carbamate; benzyl{(5S)-5-[(tert-butoxycarbonyl)amino]-6-[(4-nitrobenzyl) (2-thienylmethyl)amino]-6-oxohexyl}carbamate; benzyl{(5R)-5-[(tert-butoxycarbonyl) amino]-6-[(4-nitrobenzyl) (2-thienylmethyl)amino]-6-oxohexyl}carbamate; tert-butyl[(2R)-1-[(4-aminobenzyl) (2-thienylmethyl)amino]-6-{[(benzyloxy)-carbonyl]amino} -1-oxohexan-2-yl]carbamate; tert-butyl[(2S)-1-[(4-aminobenzyl)(2-thienylmethyl)amino]-6-{[(benzyloxy)-carbonyl]amino}-1-oxohexan-2-yl]carbamate; benzyl{(5S)-5-[(tert-butoxycarbonyl) amino]-6-[methyl(2-thienylmethyl)amino]-6-oxohexyl}carbamate; benzyl{(5S)-5-[(tert-butoxycarbonyl)amino]-6-[butyl(2-thienylmethyl)amino]-6-oxohexyl}carbamate; benzyl{(5S)-6-[bis(4-methoxybenzyl)amino]-5-[(tert-butoxycarbonyl)amino]-6-oxohexyl}carbamate; benzyl{(5S)-5-[(tert-butoxycarbonyl)amino]-6-oxo-6-[(pyridin-4-ylmethyl) (-2-thienylmethyl)amino]hexyl} carbamate; benzyl{(5S)-5-[(tert-butoxycarbonyl) amino]-6-oxo-6-[(pyridin-3-ylmethyl)(-2-thienylmethyl)amino]hexyl}carbamate; benzyl{(5S)-6-[bis(pyndin-4-ylmethyl)amino]-5-[(tert-butoxycarbonyl)amino]-6-oxohexyl}carbamate; tert-butyl{(2S)-1-[bis(2-thienylmethyl)amino]-1-oxo-6-[(2-thienylsulfonyl)amino]hexan-2-yl}carbamate; tert-butyl {(2S)-6-acetamido-1-[bis(2-thienylmethyl)amino]-1-oxohexan-2-yl}carbamate; tert-butyl{(2S)-1-[bis(2-thienylmethyl) amino]-1-oxo-6-[(trifluoroacetyl)amino]hexan-2-yl}carbamate; tert-butyl{(2S)-1-[bis(2-thienylmethyl)amino]-6-[(methylsulfonyl)amino]-1-oxohexan-2-yl}carbamate; tert-butyl{(2S)-1-[bis(2-thienylmethyl)amino]-1-oxo-6-[(2-thienylcarbonyl) amino]hexan-2-yl}carbamate; tert-butyl{(2S)-1-[bis(2-thienylmethyl)amino]-1-oxo-6-[(phenylsulfonyl)amino]hexan-2-yl}carbamate; tert-butyl{(2S)-1-[bis(2-thienylmethyl) amino]-1 -oxo-6-[(pyridin-3-ylcarbonyl)amino]hexan-2-yl}carbamate; tert-butyl{(2S)-1-[bis(2-thienylmethyl)amino]-1-oxo-6-[(2-thienylacetyl)amino]hexan-2-yl}carbamate; tert-butyl{(2S)-1-[bis(2-thienylmethyl)amino]-6-hydroxy-1-oxohexan-2-yl}carbamate; tert-butyl[(2S)-1-[bis(2-thienylmethyl)amino]-1-oxo-6-{[(trifluoromethyl)sulfonyl]amino}hexan-2-yl]c arbamate; tert-butyl{(2S)-6-[(benzylsulfonyl)amino]-1-[bis(2-thienylmethyl)amino]-1-oxohexan-2-yl}carbamate; tert-butyl{(2S)-6-[benzyl(trifluoroacetyl)amino]-1-[bis (2-thienylmethyl)amino]-1-oxohexan-2-yl}carbamate; tert-butyl[(1R)-2-[bis(2-thienylmethyl) amino]-1-(4-hydroxyphenyl)-2-oxoet- hyl]carbamate; methyl(4S)-5-[bis(2-thienylmethyl)amino]-4-[(tert-butoxycarbonyl)amino]-5-oxopentanoate; benzyl{(3S)-4-[bis(thiophen-2-ylmethyl)amino]-3-[(tert-butoxycarbonyl)amino]-4-oxobutyl}carbamate;benzyl{(4S)-5-[bis(2-thienylmethyl)amino]-4-[(tert-butoxycarbonyl)amino]-5-oxopentyl}carbamate; tert-butyl{2-[bis(2-thienylmethyl)amino]-2-oxoethyl}carbamate; tert-butyl{2-[bis(2-thienylmethyl)amino]-2-oxoethyl}methylcarbamate; N,N-bis(2-thienylmethyl)-6-[(2-thienylsulfonyl)amino]hexanamide; N-{6-[bis(2-thienylmethyl)amino]-6-oxohexyl}thiophene-2-carboxamide; N-{6-[bis(2-thienylmethyl)amino]-6-oxohexyl}-N-(2-thienylmethyl)thiophene-2-carboxamide; N-benzyl-N-{6-[bis(2-thienylmethyl)amino]-6-oxohexyl}thiophene-2-carboxamide; 6-[benzyl(2-thienylsulfonyl)amino]-N,N-bis(2-thienylmethyl)hexanamide; 6-[methyl(2-thienylsulfonyl)amino]- N,N-bis(2-thienylmethyl)hexanamide; 6-[(benzylsulfonyl)amino]-N,N-bis(2-thienylmethyl)hexanamide; 6-[(2-thienylacetyl)amino]-N,N-bis(2-thienylmethyl)hexanamide; N-{6-[bis(2-thienylmethyl)amino]-6-oxohexyl}-N-(3-methoxybenzyl)thiophene- -2-carboxamide; 6-[(3-methoxybenzyl)(2-thienylsulfonyl)amino]-N,N-bis(2-thienylmethyl)hexanamide; 6-[(benzylsulfonyl)(3-methoxybenzyl)amino]-N,N-bis(2-thienylmethyl)hexanamide; benzyl{6-[bis(2-thienylmethyl)amino]-6-oxohexyl}carbamate; tert-butyl{6-[bis(thiophen-2-ylmethyl)amino]-6-oxohexyl}carbamate; tert-butyl[(2S)-1-[bis(2-thienylmethyl)amino]-3-(4-hydroxyphenyl)-1-oxopr- opan-2-yl]carbamate; Methyl(5S)-6-[bis(2-thienylmethyl)amino]-5-[(tert-butoxycarbonyl)amino]-6-oxohexanoate; (2S)-2-[acetyl(methyl)amino]-N,N-bis(2-thienylmethyl)hexanamide; benzyl{(5S)-5-[acetyl(methyl)amino]-6-[bis(2-thienylmethyl)amino]-6-oxohexyl}carbamate; (2S)-6-{[(benzyloxy)carbonyl]amino}-1-[bis(2-thienylmethyl)amino]-1-oxohexan-2-ylacetate; tert-butyl{(2S)-6-[benzyl(2-thienylsulfonyl)amino]-1-[bis(2-thienylmethyl)amino]-1-oxohexan-2-yl}car bamate; benzyl{(5S)-6-{bis[4-(trifluoromethoxy)benzyl]amino}-5-[(tert-butoxycarbonyl)amino]-6-oxohexyl}carbamate; benzyl[(5S)-5-[(tert-butoxycarbonyl)amino]-6-oxo-6-{(2-thienylmethyl)[2-(trifluoromethyl)benzyl]amino}hexyl]carbamate; benzyl[(5S)-5-[(tert-butoxycarbonyl)amino]-6-oxo-6-{(2-thienylmethyl) [2-(trifluoromethoxy) benzyl]amino}hexyl]carbamate; benzyl[(5S)-5-[(tert-butoxycarbonyl)amino]-6-{[2-(difluoromethoxy)benzyl]-(2-thienylmethyl)amino}-6-oxohexyl]carbamate; tert-butyl{6-[bis(4-methoxybenzyl)amino]-6-oxohexyl}carbamate; N-{6-[bis(4-methoxybenzyl)amino]-6-oxohexyl}-4-methoxybenzamide; N-{6-[bis(4-methoxybenzyl)amino]-6-oxohexyl}-4-methoxy-N-(4-methoxybenzyl- )benzamide; N-{6-[bis(2-thienylmethyl)amino]-6-oxohexyl}-N-methylthiophene-2-carboxamide; 6-[(3-methoxybenzyl)(2-thienylacetyl)amino]-N,N-bis(2-thienylmethyl)h- exanamide; tert-butyl{4-[bis(2-thienylmethyl)amino]-4-oxobutyl}carbamate; methyl(3S)-3-(1,3-benzodioxol-5-yl)-3-[({4-[bis(2-thienylmethyl)amino]-4-oxobutyl} carbamoyl)amino]propanoate; 6-{[(3-chloropropyl)sulfonyl]amino}-N,N-bis (4-methoxybenzyl)hexanamide; 6-(1,1-dioxido-1,2-thiazolidin-2-yl)-N,N-bis (4-methoxybenzyl)hexanamide; N,N-bis(4-methoxybenzyl)-6-({ [2-(morpholin-4-yl)ethyl] sulfonyl} amino)hexanamide; 3-{[bis(2-thienylmethyl)carbamoyl]amino}-N,N-bis(2-thienylmethyl)propanamide; tert-butyl{3-[bis(2-thienylmethyl)amino]-3-oxopropyl} butylcarbamate; 3-{[bis(2-thienylmethyl)carbamoyl](butyl)amino}-N,N-bis (2-thienylmethyl)propanamide; 3-{butyl[(2-thienylmethyl)carbamoyl]amino}-N,N-bis (2-thienylmethyl)propanamide; 4-(1,1-dioxido-1,2-thiazolidin-2-yl)-N,N-bis (2-thienylmethyl)butanamide; N,N-bis(2-thienylmethyl)-3-{[(2-thienylmethyl)carbamoyl]amino} propanamide; benzyl{(5S)-6-[bis(2-thienylmethyl)amino]-5-hydroxy-6-oxohexyl}carbamate; benzyl{(5S)-6-[bis(2-thienylmethyl)amino]-5-cyano-6-oxohexyl}carbamate; benzyl{(5R)-5-azido-6-[bis(2-thienylmethyl)amino]-6-oxohexy1}carbamate; S-{(2R)-6-{[(benzyloxy)carbonyl]amino}-1-[bis(2-thienylmethyl)amino]-1-oxohexan-2-yl}ethanethioate; tert-butyl[(2S)-1-[bis(2-thienylmethyl)amino]-6-({[(4-bromobenzyl)oxy]carbonyl}amino)-1-oxohexan-2-yl]carbamate; 4-azidobenzyl{(5S)-6-[bis(2-thienylmethyl)amino]-5-[(tert-butoxycarbonyl)-amino]-6-oxohexyl}carbamate; benzyl{(5S)-6-[(4-bromobenzyl)(2-thienylmethyl)amino]-5-[(tert-butoxycarbonyl)amino]-6-oxohexyl}carbamate; tert-butyl[(2S)-1-[(4-azidobenzyl)(2-thienylmethyl)amino]-6-{[(benzyloxy)-carbonyl]amino} -1-oxohexan-2-yl]carbamate; tert-butyl{(2S)- 1-[(4-bromobenzyl)(2-thienylmethyl)amino]-1-oxohexan-2-yl}carbamate; benzyl{(5S)-6-[bis(3-thienylmethyl)amino]-5-[(tert-butoxycarbonyl) amino]-6-oxohexyl}carbamate; benzyl{(5S)-5-[(tert-butoxycarbonyl)amino]-6-[(cyclopropylmethyl)(2-thienylmethyl)amino]-6-oxohexyl}carbamate, and mixtures or combinations thereof.

In some embodiments, a compound is selected from the group consisting of methyl(2S)-6-{[(benzyloxy)carbonyl]amino}-2-[benzyl(2-thienylsulfonyl)amino]hexanoate; methyl(2S)-6-{[(benzyloxy)carbonyl]amino}-2-[benzyl(phenylsulfonyl)amino]hexanoate; methyl(2S)-6-{[(benzyloxy)carbonyl]amino}-2-[(2-thienylcarbonyl-)(2-thienylmethyl)amino]hexanoate ; methyl(2S)-6-{[(benzyloxy)carbonyl]amino}-2-[(2-thienylacetyl)(2-thienylm-ethyl)amino]hexanoate; methyl(2S)-2-[benzyl(isobutylsulfonyl)amino]-6-{[(benzyloxy) carbonyl]amino}hexanoate; benzyl{(5S)-5-[(tert-butoxycarbonyl)amino]-6-[(2-thienylmethyl)(2-thienylsulfonyl)amino]hexyl}carbamate; benzyl{(5S)-5-[(tert-butoxycarbonyl)amino]-6-[(2-thienylacetyl)(2-thienylmethyl)amino]hexyl}carbamate; benzyl{(5S)-5-[(tert-butoxycarbonyl)amino]-6-[(methylsulfonyl)(2-thienylmethyl)amino]hexyl}carbam ate; benzyl{(5S)-5-[(tert-butoxycarbonyl)amino]-6-[(phenylsulfonyl)(2-thienylmethyl)amino]hexyl}carbamate; benzyl{(5S)-5-[(tert-butoxycarbonyl)amino]-6-[(2-thienylcarbonyl)(2-thienylmethyl)amino]hexyl}carbamate; N,N'-heptane-1,7-diylbis[N-(2-thienylmethyl)benzamide]; N,N'-heptane-1,7-diylbis[N-(2-thienylmethyl) thiophene-2-carboxamide]; benzyl[(5 S)-5-[(tert-butoxycarbonyl)amino]-6-{[(4-methoxyphenyl)sulfonyl]-(2-thienylmethyl)amino}hexyl]carbamate; benzyl{(5S)-5-[(tert-butoxycarbonyl)amino]-6-[(4-methoxybenzoyl)(2-thienylmethyl)amino]hexyl} carbamate; N,N'-hexane-1,6-diylbis[N-(2-thienylmethyl) thiophene-2-carboxamide]; N,N'-hexane-1,6-diylbis[N-(3-methoxybenzyl) thiophene-2-carboxamide]; tert-butyl{5-[(4-methoxybenzyl)(2-thienylsulfonyl) amino]pentyl } carbamate; N,N'-pentane-1,5-diylbis[N-(3-methoxybenzyl)thiophene-2-sulfonamide]; N-(3-methoxybenzyl)-N-{5-[(2-thienylsulfonyl)amino]pentyl}thiophene-2-sulfonamide; tert-butyl{5-[(2-thienylcarbonyl)(2-thienylmethyl)amino]pentyl} carbamate; N-(3-methoxybenzyl)-N-{5-[(2-thienylcarbonyl)amino]pentyl}thiophene-2-carboxamide; N,N'-pentane-1,5-diylbis[N-(3-methoxybenzyl)thiophene-2-carboxamide]; and mixtures or combinations thereof.

In some embodiments, a compound is selected from the group consisting of N,N,N',N'-tetrakis(2-thienylmethyl)pentanediamide; N-(3-methoxybenzyl)-N,N',N'-tris(2-thienylmethyl)pentanediamide; N,N,N'-tris(2-thienylmethyl)pentanediamide; N'-[2-(2-thienyl)ethyl]-N,N-bis(2-thienylmethyl)pentanediamide; N-[2-(2-thienyl)ethyl]-N,N',N'-tris (2-thienylmethyl)pentanediamide; N,N-bis(pyridin-4-ylmethyl)-N',N'-bis(2-thienylmethyl) pentanediamide; N,N-bis(pyridin-3-ylmethyl)-N',N'-bis(2-thienylmethyl)pentanediamide; N,N-bis(3-methoxybenzyl)-N',N'-bis(2-thienylmethyl)pentanediamide; N,N,N',N'-tetrakis (4-methoxybenzyl)pentanediamide; N,N,N',N'-tetrakis(2-thienylmethyl)hexanediamide; N,N,N',N'-tetrakis(4-methoxybenzyl)hexanediamide; N,N,N',N'-tetrakis(3-methoxybenzyl) hexanediamide; N,N,N',N'-tetrakis(2-thienylmethyl)heptanediamide; 2,2'-(1,3-phenylene)bis[N,N-bis (2-thienylmethyl)acetamide]; N,N,N',N'-tetrakis(4-methoxybenzyl)heptanediamide; N,N,N',N'-tetrakis(2-thienylmethyl)octanediamide; (3E)-N,N,N',N'-tetrakis(2-thienylmethyl) hex-3-enediamide; 2,2'-oxybis[N,N-bis(2-thienylmethyl)acetamide]; 3-oxo-1-(2-thienyl)-2-(2-thienylmethyl)-4,7,10-trioxa-2-azadodecan-12-yl bis(2-thienylmethyl)carbamate; N,N,N',N'-tetrakis(4-methoxybenzyl)succinamideethane-1,2-diyl bis[bis(2-thienylmethyl)carbamate]; N,N,N',N'-tetrakis(4-methoxybenzyl)octanediamide; N,N,N',N'-tetrakis(2-thienylmethyl)pyridine-3,5-dicarboxamide; N,N,N',N'-tetrakis(2-thienylmethyl) pyridine-2,6-dicarboxamide; N,N,N',N'-tetrakis(2-thienylmethyl)pyridine-2,4-dicarboxamide; 2,2'-(1,4-phenylene)bis[N,N-bis(2-thienylmethyl)acetamide]; 8-{2-[bis(2-thienylmethyl)amino]-2-oxoethoxy}-N,N-bis(2-thienylmethyl)quinoline-2-carboxamide; N,N'-bis(4-methoxybenzyl)-N,N'-bis(2-thienylmethyl)hexanediamide; tert-butyl{(2S)-1,6-bis[bis(2-thienylmethyl)amino] -1,6-dioxohexan-2-yl}carbamate ; and mixtures or combinations thereof.

In some embodiments, a compound is selected from the group consisting of N-{2-[bis(2-thienylmethyl)sulfamoyl]ethyl}-N-(2-thienylmethyl)thiophene-2-sulfonamide; N-{2-[bis(2-thienylmethyl)sulfamoyl]ethyl}-N-(2-thienylmethyl)thiophene-2-carboxamide; 2-{butyl[(2-thienylmethyl)carbamoyl]amino}-N,N-bis(2-thienylmethyl)ethanesulfonamide; 2-{[bis(2-thienylmethyl)carbamoyl](butyl)amino}-N,N-bis(2-thienylmethyl)ethanesulfonamide; N-{3-[bis(2-thienylmethyl)sulfamoyl]propyl}-N-(2-thienylmethyl)thiophene-2-sulfonamide; 2-[(methylsulfonyl)(2-thienylmethyl)amino]-N,N-bis(2-thienylmethyl)ethanesulfonamide; 2-{[bis(2-thienylmethyl)carbamoyl]amino}-N,N-bis(2-thienylmethyl)ethanesulfonamide; N-{2-[bis(2-thienylmethyl)sulfamoyl]ethyl}thiophene-2-sulfonamide; N-{2-[bis(2-thienylmethyl)sulfamoyl]ethyl}-2-(2-thienyl)acetamide; N-{2-[bis(2-thienylmethyl)sulfamoyl]ethyl}thiophene-2-carboxamide; N,N-bis(2-thienylmethyl)-2-{[(2-thienylmethyl)carbamoyl]amino}ethanesulfonamide; 2-({2-[bis(2-thienylmethyl)sulfamoyl]ethyl}amino)-N,N-bis(2-thienylmethyl)acetamide; 3-[{2-[bis(2-thienylmethyl)amino]-2-oxoethyl}(butyl)amino]-N,N-bis(2-thienylmethyl)propanamide; 2-[{2-[bis(2-thienylmethyl)sulfamoyl]ethyl}(methyl)amino]-N,N-bis(2-thien ylmethyl)acetamide; 2-[{2-[bis(2-thienylmethyl)sulfamoyl]ethyl}(butyl)amino]-N,N-bis(2-thienylmethyl)acetamide; 3-({2-[bis(2-thienylmethyl)sulfamoyl]ethyl}amino)-N,N-bis(2-thienylmethyl)propanamide; 3-({2-[bis(2-thienylmethyl)sulfamoyl]ethyl}amino)-N,N-bis(4-methoxybenzyl)propanamide; 3-({2-[bis(4-methoxybenzyl)sulfamoyl]ethyl}amino)-N,N-bis(2-thienylmethyl)propanamide; 3-[{2-[bis(2-thienylmethyl)sulfamoyl]ethyl}(methyl)amino]-N,N-bis(2-thienylmethyl)propanamide; 3-[{2-[bis(4-methoxybenzyl)sulfamoyl]ethyl}(methyl)amino]-N,N-bis(2-thienylmethyl) propanamide; (2S)-2-({2-[bis(2-thienylmethyl)sulfamoyl]ethyl}amino)- N,N-bis(2-thienylmethyl)hexanamide; (2S)-2-({2-[bis(4-methoxybenzyl)sulfamoyl]ethyl}amino)- N,N-bis(2-thienylmethyl)hexanamide; 2-(acetyl{2-[bis(2-thienylmethyl)sulfamoyl]ethyl}amino)- N,N-bis(2-thienylmethyl)acetamide; 2-(acetyl{2-[bis(4-methoxybenzyl)sulfamoyl]ethyl}amino)- N,N-bis(2-thienyl-methyl)acetamide; and mixtures or combinations thereof.

In some embodiments, a compound is selected from the group consisting of tert-butyl[(2S)-1-{[bis(cyclopropylmethyl)carbamoyl]oxy}hexan-2-yl]carbamate; (2S)-2-[(tert-butoxycarbonyl)amino]hexyldiisobutylcarbamate; methyl(8S,12S)-12-(1,3-benzodioxol-5-yl)-8-butyl-4-isobutyl-2-methyl-5,10--dioxo-6-oxa-4,9,11-triaz atetradecan-14-oate; benzyl{(5S)-6-[bis(cyclopropylmethyl)amino]-5-[(tert-butoxycarbonyl)amino-]-6-oxohexyl}carbamate; and mixtures or combinations thereof.

In accordance with certain embodiments, a pharmaceutical composition is provided comprising an above-described compound or a pharmaceutically acceptable salt thereof; and a pharmaceutically acceptable carrier.

Also disclosed herein is a method of treating integrin-expressing cells. The integrin may be one or more of α4β1, α5β1, α4β7, αvβ3 and αLβ2, for example. The method of treating integrin-expressing cells may comprise contacting at least one integrin-expressing cell in vitro with an agonist of said integrin, wherein said agonist is a compound having the general formula (I), where **R¹** and **R²** are independently selected from the group consisting of alkyl, aryl, aralkyl, cycloalkyl, cycloalkylalkyl, haloalkyl, heterocylcyl and heterocyclylalkyl, one of **M¹** and **M²** is CO or SO₂ and the other is (C**R⁴R⁵**)ₗ, provided that when M² is CO, **M³** is O, S, N**R⁶** or (C**R⁷R⁸**)ₘ, and provided that when **M²** is SO₂ or (C**R⁴R⁵**)ₗ, **M³** is (C**R⁷R⁸**)_{m,} **M⁴** is absent or (C**R⁹R¹⁰**)ₙ, **M⁵** is absent or is O or (C**R¹¹R¹²**)ₚ, **M⁶** is absent or is selected from the group consisting of (CH₂)_{q}, (CH₂)_{q}BCH=CHB(CH₂)ᵣ, (CH₂)_{q}BaryleneB(CH₂)ᵣ, (CH₂CH₂O)_{q}, and N**R³⁴**(CH₂)_{q}, and **R³** is selected from the group consisting of hydrogen, OH, O**R¹⁶**, CON**R¹³R¹⁴**, N**R¹⁵**COO**R¹⁶**, N**R¹⁵**CO**R¹⁶**, N**R¹⁵**CON**R¹³R¹⁴**, N**R¹⁵**SO₂**R¹⁶**, OCO**R¹⁶**, COO**R¹⁶**, alkyl, aryl, aralkyl, S**R¹⁶**, heterocyclyl, hydroxyalkyl and guanadino, **R³⁴**, when present, is selected form the group consisting of alkyl, aralkyl, CO**R³⁵**, and SO₂**R³⁵**, **R³⁵**, when present, is selected form the group consisting of alkyl, aryl, and aralkyl, and **R¹²**, when present, is selected from the group consisting of hydrogen, alkyl, OH, N₃, CN, N**R²¹**CON**R²²R²³**, N**R²¹**CO**R²⁴**, N**R²¹**COO**R²⁴**, N**R²¹**SO₂**R²⁴**, CON**R²²R²³**, COOR²⁴, OCO**R²⁴**, OR²⁴, SCO**R²⁴**, SR²⁴, azido, CN, and O(CH₂CH₂O)ₛR²⁴, **R⁴**, **R⁵**, **R⁶**, **R⁷**, **R⁸**, **R⁹**, R¹⁰, **R¹¹, R¹⁵**, and **R²¹**, each of which when present, is independently selected from the group consisting of hydrogen, lower alkyl and aralkyl, R¹³, R¹⁴, **R¹⁶**, R²², **R²³** and **R²⁴**, each of which when present, is independently selected from the group consisting of alkyl, aryl, aralkyl, cycloalkyl, cycloalkylalkyl, haloalkyl, heterocyclyl, heterocyclylalkyl, alkoxycarbonyl, alkoxycarbonylalkyl, 1, m, n and p are independently integers from 0 to 1, q, r and s are independently integers from 0 to 6, **R¹, R²**, R³, **R⁴**, **R⁵**, **R⁶**, **R⁷**, **R⁸**, **R⁹**, **R¹⁰**, **R¹¹**, **R¹²**, **R¹³**, **R¹⁴**, **R¹⁵**, **R¹⁶**, **R²¹**, **R²²**, **R²³**, **R²⁴**, **R³⁴** and **R³⁵**, each of which when present, is independently either unsubstituted or substituted with one or more substituents selected from the group consisting of alkyl, aryl, aralkyl, cycloalkyl, cycloalkylalkyl, heterocyclyl, heterocyclylalkyl, heterocyclylaryl, hydroxyl, alkoxy, haloalkoxy, azido, hydroxyalkyl, aryloxy, hydroxyaryl, alkoxyaryl, halo, haloalkyl, haloaryl, amino, alkylamino, dialkylamino, arylamino, diarylamino, BNHCO(alkyl), BNHCO(aryl), BNHCO(haloalkyl), BNHSO₂(alkyl), BNHSO₂(aryl), alkoxycarbonyl, alkoxycarbonylalkyl, BOCO(alkylamino), BOCO(dialkylamino), and mixtures or combinations thereof.

Also disclosed is a method of enhancing binding of cells to an integrin-binding ligand, wherein the method comprises treating integrin-expressing cells in vitro with an agonist of integrin described above, wherein said integrin is selected from the group consisting of α4β1, α5β1, α4β7, αvβ3 and αLβ2; and contacting the treated cells with an integrin-binding ligand.

The agonist of integrin utilized in an above described method may be a compound selected from the group consisting of methyl(3R)-3-(1,3-benzodioxol-5-yl)-3-[({(2R)-1-[bis(2-thienylmethyl)amino]-1-oxohexan-2-yl}carbamoyl)amino]propanoate; methyl(3S)-3-(1,3-benzodioxol-5-yl)-3-[({(2R)-6-{[(benzyloxy)carbonyl]amino}-1-[bis(thiophen-2-yl methyl)amino]-1-oxohexan-2-yl}carbamoyl)amino]propanoate; methyl(3S)-3-(1,3-benzodioxol-5-yl)-3-[({(2R)-1-[bis(thiophen-2-yl-methyl)amino]-1-oxohexan-2-yl} carbamoyl)amino]propanoate; methyl(3R)-3-(1,3-benzodioxol-5-yl)-3-[({(2S)-1-[bis(2-thienylmethyl)amino]-1-oxohexan-2-yl}carbamoyl)amino]propanoate; methyl(3S)-3-(1,3-benzodioxol-5-yl)-3-[({2-[bis(2-thienylmethyl)amino]-2-oxoethyl}carbamoyl)amino ]propanoate; methyl(3S)-3-(1,3-benzodioxol-5-yl)- 3-[({2-[bis(2-thienylmethyl)amino]-2-oxoethyl}carbamoyl)amino]propanoate; methyl(3S)-3-(1,3-benzodioxol-5-yl)-3-{[{2-[bis (2-thienylmethyl)amino]-2-oxoethyl}(methyl)carbamoyl]amino}propanoate; methyl(3R)-3-(1,3-benzodioxol-5-yl)- 3-{[{2-[bis(2-thienylmethyl)amino]-2-oxoethyl} (methyl)carbamoyl]amino}propanoate; methyl(3R)-3-(1,3-benzodioxol-5-yl)-3-[({2-[bis (2-thienylmethyl)amino]-2-oxoethyl}carbamoyl)amino]propanoate; methyl(2R)-[({(2S)-1-[bis (thiophen-2-ylmethyl)amino]-1-oxohexan-2-yl}carbamoyl)amino](phenyl)ethanoate; methyl 3-[({(2S)-1-[bis(2-thienylmethyl)amino]-1-oxohexan-2-yl}carbamoyl)amino]propanoate; (2S)-2-[(isopropylcarbamoyl)amino]-N,N-bis(2-thienylmethyl)hexanamide; (2S)-2-[(methylcarbamoyl)amino]-N,N-bis(2-thienylmethyl)hexanamide; (2S)-2-[(benzylcarbamoyl)amino]-N,N-bis(2-thienylmethyl)hexanamide; (2R)-2-[(benzylcarbamoyl)amino]-N,N-bis(2-thienylmethyl)hexanamide; benzyl{(5S)-5-[(benzylcarbamoyl)amino]-6-[bis(2-thienylmethyl)amino]-6-ox- ohexyl}carbamate; (2S)-2-{[(1,3-benzodioxol-5-ylmethyl)carbamoyl]amino}-N,N-bis(2-thienylme- thyl)hexanamide; benzyl[(5S)-6-[bis(2-thienylmethyl)amino]-6-oxo-5-{[(pyridin-3-ylmethyl)carbamoyl]amino} hexyl]carbamate; (2S)-2-{[(pyridin-3-ylmethyl)carbamoyl]amino}-N,N-bis(2-thienylmethyl) hexanamide; (2S)-2-({[(6-methoxypyridin-3-yl)methyl]carbamoyl}amino)-N,N-bis(-2-thienylmethyl) hexanamide; (2S)-2-({[3-(morpholin-4-yl)benzyl]carbamoyl}amino)-N,N-bis(2-thienylmethyl)hexanamide; (2S)-2-{[(4-hydroxybenzyl)carbamoyl]amino}-N,N-bis(2-thienylmethyl)hexanamide; (2S)-2-({[4-(dimethylamino)benzyl]carbamoyl}amino)-N,N-bis(2-thienylmethyl)hexanamide; benzyl[(5S)-6-[bis(2-thienylmethyl)amino]-5-({[3-(morpholin-4-yl)benzyl]carbamoyl}amino)-6-oxohexyl]carbamate; benzyl{(5S)-6-[bis(2-thienylmethyl)amino]-5-[({3-[(methylsulfonyl)amino]benzyl}carbamoyl) amino]-6-oxohexyl}carbamate; benzyl{(2S)-6-{[(benzyloxy)carbonyl]amino}-1-[bis(2-thienylmethyl)amino]-1-oxohexan-2-yl}carbamate; benzyl{(2S)-1-[bis(2-thienylmethyl)amino]-1-oxohexan-2-yl}carbamate; benzyl{(5S)-6-[bis(2-thienylmethyl) amino]-5-[(ethoxycarbonyl)amino]-6-oxohexyl}carbamate; benzyl[(5S)-6-[bis(2-thienylmethyl) amino]-5-(butyrylamino)-6-oxohexyl]carbamate; benzyl{(5S)-6-[bis(2-thienylmethyl)amino]-6-oxo-5-[(3-phenoxypropanoyl)amino]hexyl}carbamate; and mixtures or combinations thereof.

Also disclosed herein is an integrin agonist used in a method of enhancing binding of cells to an integrin-binding ligand which is selected from the group consisting of compounds having the

general formula (I)

where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein **q** is an integer from 1 to 6, and
**R³** is CON**R¹³R¹⁴**, where **R¹³** and **R¹⁴** are independently aralkyl.

In accordance with some further embodiments, an integrin agonist used in a method of enhancing binding of cells to an integrin-binding ligand is selected from the group consisting of N-bis(2-thienylmethyl)benzenesulfonamide; N,N-bis(2-thienylmethyl)acetamide; 1-phenyl-N,N-bis(2-thienylmethyl)methanesulfonamide; 2-methyl-N,N-bis(2-thienylmethyl) propane-1-sulfonamide; N-(3-methoxybenzyl)-N-(2-thienylmethyl)benzenesulfonamide; N-(3-methoxybenzyl)-N-(2-thienylmethyl)propane-2-sulfonamide; N-(3-methoxybenzyl)-2-methyl-B (2-thienylmethyl)propane-1-sulfonamide; N-(4-hydroxybenzyl)-3-methoxy-N-(2-thienylmethyl) benzenesulfonamide; N-[2-(2-thienyl)ethyl]-N-(2-thienylmethyl)benzenesulfonamide; N,N-dibenzylbenzenesulfonamide; N-(pyridin-3-ylmethyl)-N-(2-thienylmethyl)benzenesulfonamide; N-butyl-N-(2-thienylmethyl)benzenesulfonamide; N-(3-hydroxypropyl)-N-(2-thienylmethyl) benzenesulfonamide; N-(2-methoxyethyl)-N-(2-thienylmethyl)benzenesulfonamide; N-(2-methoxyethyl)-N-(2-thienylmethyl)thiophene-2-sulfonamide; N,N-bis(3-methoxybenzyl)benzenesulfonamide; N,N-bis(4-methoxybenzyl) thiophene-2-sulfonamide; 2-chloro-N,N-bis(2-thienylmethyl)benzenesulfonamide; 3-chloro-N,N-bis(2-thienylmethyl)benzenesulfonamide; 4-chloro-N,N-bis(2-thienylmethyl) benzenesulfonamide; 3-methoxy-N,N-bis(2-thienylmethyl)benzenesulfonamide; 4-methoxy-N,N-bis(2-thienylmethyl)benzenesulfonamide; N,N-bis(pyridin-4-ylmethyl )benzenesulfonamide; N,N-bis(pyridin-3-ylmethyl)benzenesulfonamide; N-(2-furylmethyl)-N-(2-thienylmethyl)benzenesulfonamide; N,N-bis(2-furylmethyl) benzenesulfonamide; N,N-bis(3 -methoxybenzyl)thiophene-2-sulfonamide; methyl 3-[bis(3-methoxybenzyl)sulfamoyl]thiophene-2-carboxylate; 2-(hydroxymethyl)-N,N-bis (3-methoxybenzyl)thiophene-3-sulfonamide; N,N-bis(4-methoxybenzyl)-3-methylbenzenesulfonamide; N-phenyl-N-(2-thienylmethyl)benzenesulfonamide; N-phenyl-N-(2-thienylmethyl)thiophene-2-sulfonamide; N-(3-methoxybenzyl)-B phenylthiophene-2-sulfonamide; N-(3-methoxybenzyl)-N-phenylbenzenesulfonamide; 3-(4-methoxyphenoxy)-N,N-bis(2-thienylmethyl)propane-1-sulfonamide; 4-methyl-N,N-bis(2-thienylmethyl)benzenesulfonamide; 2-methyl-N,N-bis(2-thienylmethyl) benzenesulfonamide; 3-methyl-N,N-bis(2-thienylmethyl)benzenesulfonamide; and mixtures or combinations thereof.

Also disclosed herein is a method of enhancing binding of cells to an integrin-binding ligand, wherein said agonist of integrin is a compound selected from the group consisting of methyl (6S,10S)-10-(1,3-benzodioxol-5-yl)-6-butyl-3,8-dioxo-1-(2-thienyl)-2-(2-thienylmethyl)-4-oxa-2,7,9-triazadodecan-12-oate; methyl(6S,10S)-10-(1,3-benzodioxol-5-yl)- 6-butyl-7-methyl-3,8-dioxo-1-(2-thienyl)-2-(2-thienylmethyl)-4-oxa-2,7,9-triazadodecan-12-oate; tert-butyl[(2S)-1-{[bis(2-thienylmethyl)carbamoyl]oxy}hexan-2-yl]carbamate; (2S)-2-{[(l,3-benzodioxol-5-ylmethyl)carbamoyl]amino}hexyl-bis(2-thienylmethyl)carbamate; methyl(6S,100S)-6-butyl-3,8-dioxo-10-phenyl-1-(2-thienyl)-2-(2-thienylmethyl)-4-oxa-2,7,9-triazadod ecan-12-oate; (2S)-2-[(benzylcarbamoyl)amino]hexyl bis(2-thienylmethyl) carbamate; (2S)-2-({[(1S)-1-(1,3-benzodioxol-5-yl)-3-hydroxypropyl]carbamoyl}amino)hexyl bis(2-thienylmethyl)carbamate; methyl(6S,100R)-10-(1,3-benzodioxol-5-yl)-6-butyl-3,8-dioxo-1-(2-thienyl)-2-(2-thienylmethyl)-4-oxa-2,7,9-triazadodecan-12-oate; benzyl{(5S)-6-{[bis(2-thienylmethyl)carbamoyl]oxy}-5-[(tert-butoxycarbonyl)amino]hexyl}carbamate; methyl(9S,13S)-13-(1,3-benzodioxol-5-yl)-9-({[bis(2-thienylmethyl)carbamoyl]oxy}methyl)-3,11-dioxo-1-phenyl-2-oxa-4,10,12-triazapentadecan -15-oate; tert-butyl[(2R)-1-{[bis(2-thienylmethyl)carbamoyl]oxy}hexan-2-yl]carbamate; tert-butyl{[bis(2-thienylmethyl)carbamoyl](butyl)amino}acetate; benzyl{(5S)-6-{[bis(4-methoxybenzyl)carbamoyl]oxy}-5-[(tert-butoxycarbonyl)amino]hexyl} carbamate; tert-butyl[(2S)-1-{[bis(4-methoxybenzyl)carbamoyl]oxy}hexan-2-yl]carbamate; methyl(6S,10S)-10-(1,3-benzodioxol-5-yl)-6-butyl-2-(4-methoxybenzyl)-1-(4-methoxyphenyl)-3,8-dio xo-4-oxa-2,7,9-triazadodecan-12-oate; (2S)-2-({[(1S)-1-(1,3-benzodioxol-5-yl)-3-hydroxypropyl]carbamoyl}amino)hexyl-bis(4-methoxybenzyl)carbamate; (2S)-2-[(tert-butoxycarbonyl)amino]hexyldibenzylcarbamate; methyl(6S,10S)-10-(1,3-benzodioxol-5-yl)-2-benzyl-6-butyl-3,8-dioxo-1-phe-nyl-4-oxa-2,7,9-triazadodecan-12-oate; tert-butyl[(2S)-1-{[bis(4-methylbenzyl)carbamoyl]oxy}hexan-2-yl]carbamate-methyl(6S,10S)-10-(1,3-benzodioxol-5-yl)-6-butyl-2-(4-methylbenzyl)-1-(4-methylphenyl)-3,8-dioxo-4-oxa-2,7,9-triazadodeca n-12-oate; tert-butyl[(2S)-1-{[bis(4-chlorobenzyl)carbamoyl]oxy}hexan-2-yl]carbamate; methyl(6S,10S)-10-(1,3-benzodioxol-5-yl)-6-butyl-2-(4-chlorobenzyl)-1-(4--chlorophenyl)-3,8-dioxo-4-oxa-2,7,9-triazadodecan-12-oate; methyl(6S,10S)-10-(1,3-benzodioxol-5-yl)-2-(4-bromobenzyl)-6-butyl-3,8-dioxo-1-(2-thienyl)-4-oxa-2,7,9-triazadodecan-12-oate; methyl(6S,10S)-2-(4-azidoobenzyl)-10-(1,3-benzodioxol-5-yl)-6-butyl-3,8-d-ioxo-1-(2-thienyl)-4-oxa-2,7,9-triazadodecan-12-oate; (2S)-2-[(tert-butoxycarbonyl)amino]hexyl phenyl(2-thienylmethyl)carbamate; methyl(6S,10S)-10-(1,3-benzodioxol-5-yl)-6-butyl-3,8-dioxo-2-phenyl-1-(2-thienyl)-4-oxa-2,7,9-triazadodecan-12-oate; tert-butyl[(2S)-1-{[bis(3-thienylmethyl)carbamoyl]oxy}hexan-2-yl]carbamate; methyl(6S,10S)-10-(1,3-benzodioxol-5-yl)-6-butyl-3,8-dioxo-1-(3-thienyl)-2-(3-thienylmethyl)-4-oxa-2,7,9-triazadodecan-12-oate; benzyl[(5S)-5-[(tert-butoxycarbonyl)amino] - 6-{[butyl(2-thienylmethyl)carbamoyl]oxy}hexyl]carbamate; (2S)-2-[(tert-butoxycarbonyl) amino]hexylbutyl(2-thienylmethyl)carbamate; methyl(3S,7S)-3-(1,3-benzodioxol-5-yl)-7-butyl-5,10-dioxo-11-(2-thienylmethyl)-9-oxa-4,6,11-triazapentadecan-1-oate; benzyl[(5S)-5-[(tert-butoxycarbonyl)amino]-6-{[(2-methoxyethyl)(2-thienylmethyl)carbamoyl]oxy}hexyl]carbamate; (2S)-2-{[(4-bromobenzyl)carbamoyl]amino}hexyl bis(2-thienylmethyl)carbamate; (2S)-2-{[(4-azidobenzyl)carbamoyl]amino}hexyl bis(2-thienylmethyl)carbamate; tert-butyl[(2S)-1-{[bis(2-thienylmethyl)carbamoyl]thio} hexan-2-yl]carbamate; methyl(6S,10S)-10-(1,3-benzodioxol-5-yl)-6-butyl- 3,8-dioxo-1-(2-thienyl)-2-(2-thienylmethyl)-4-thia-2,7,9-triazadodecan-12-oate; and mixtures or combinations thereof.

Also disclosed herein is a method of enhancing binding of cells to an integrin-binding ligand, wherein an agonist of integrin is a compound selected from the group consisting of benzyl{(5R)-5-[(tert-butoxycarbonyl)amino]-6-[(3-methoxybenzyl)(2-thienylmethyl)amino]-6-oxohexy l}carbamate; benzyl{(5R)-6-[bis(3-methoxybenzyl)amino]- 5-[(tert-butoxycarbonyl)amino] -6-oxohexyl}carbamate; benzyl{(5S)-6-[bis(4-methoxybenzyl)amino]-5-[(tert-butoxycarbonyl) amino]-6-oxohexyl} carbamate; benzyl {(5S)-5-[(tert-butoxycarbonyl)amino]-6-oxo-6-[(pyridin-3-ylmethyl)(-2-thienylmethyl)amino]hexyl}carbamate; benzyl{(5S)-5-[(tert-butoxycarbonyl)amino]-6-oxo-6-[(pyridin-4-ylmethyl)(2-thienylmethyl)amino]hexyl}carbamate; (2S)-2-[methyl(phenylsulfonyl)amino]-N,N-bis (2-thienylmethyl)hexanamide; (2S)-2-({[3-(4-methoxyphenoxy)propyl]sulfonyl}amino)-N,N-bis (2-thienylmet- hyl)hexanamide; benzyl{(5R)-6-[bis(2-thienylmethyl)amino]-5-[(tert-butoxycarbonyl) amino]-6-oxohexyl}carbamate; benzyl{(5S)-6-[bis(2-thienylmethyl)amino]-5-[(tert-butoxycarbonyl) amino]-6-oxohexyl}carbamate; benzyl{(5S)-6-[bis(2-thienylmethyl)amino]-6-oxo-5- [(2-thienylsulfonyl)amino]hexyl}carbamate; tert-butyl{(2S)-1-[bis(2-thienylmethyl)amino]-1-oxo-6-[(2-thienylsulfonyl)amino]hexan-2-yl}carbamate; 6-[methyl(2-thienylsulfonyl)amino]-N,N-bis (2-thienylmethyl)hexanamide; 6-[(2-thienylacetyl)amino]-N,N-bis(2-thienylmethyl)hexanamide; benzyl(4S)-5-[bis(2-thienylmethyl)amino]-4-[(tert-butoxycarbonyl)amino]-5-oxopentyl}carbamate; benzyl[(5S)-5-[(tert-butoxycarbonyl)amino]-6-oxo-6-{(2-thienylmethyl)[2-(trifluoromethyl)benzyl]ami no}hexyl]carbamate; benzyl[(5S)-5-[(tert-butoxycarbonyl)amino]-6-oxo-6-{(2-thienylmethyl) [2-(trifluoromethoxy)benzyl]amino}hexyl]carbamate; benzyl[(5S)-5-[(tert-butoxycarbonyl)amino]-6-{[2-(difluoromethoxy)benzyl]-(2-thienylmethyl)amino}-6-oxohexyl]carbamate; tert-butyl{6-[bis(4-methoxybenzyl)amino]-6-oxohexyl}carbamate; N-{6-[bis(4-methoxybenzyl) amino]-6-oxohexyl}-4-methoxy-N-(4-methoxybenzyl)benzamide; N-{6-[bis(2-thienylmethyl) amino]-6-oxohexyl}-N-methylthiophene-2-carboxamide; 6-[(3-methoxybenzyl) (2-thienylacetyl)amino]-N,N-bis(2-thienylmethyl)hexanamide; methyl(3S)-3-(1,3-benzodioxol-5-yl)-3-[({4-[bis(2-thienylmethyl)amino]-4-oxobutyl}carbamoyl)amino]propanoate; 6-{[(3-chloropropyl)sulfonyl]amino}-N,N-bis(4-methoxybenzyl)hexanamide; 3-{[bis(2-thienylmethyl)carbamoyl]amino}-N,N-bis(2-thienylmethyl)propanamide; 3-{butyl[(2-thienylmethyl)carbamoyl]amino}-N,N-bis(2-thienylmethyl)propanamide; benzyl{(5S)-6-[bis(2-thienylmethyl)amino]-5-cyano-6-oxohexyl}carbamate; benzyl{(5R)-5-azido-6-[bis(2-thienylmethyl)amino]-6-oxohexyl}carbamate; benzyl{(5S)-6-[bis(3-thienylmethyl)amino]-5-[(tert-butoxycarbon-yl)amino]-6-oxohexyl}carbamate; and mixtures or combinations thereof.

Also disclosed herein is a method of enhancing binding of cells to an integrin-binding ligand, wherein an agonist of integrin is a compound selected from the group consisting of N-(3-methoxybenzyl)-N,N',N'-tris(2-thienylmethyl)pentanediamide; N-[2-(2-thienyl)ethyl]-N,N',N'-tris(2-thienylmethyl)pentanediamide; N,N-bis(3-methoxybenzyl)-N',N'-bis (2-thienylmethyl)pentanediamide; N,N-bis(pyridin-4-ylmethyl)-N',N'-bis (2-thienylmethyl)pentanediamide; N,N,N',N'-tetrakis(2-thienylmethyl)hexanediamide; N,N,N',N'-tetrakis(3-methoxybenzyl)hexanediamide; N,N,N',N'-tetrakis(4-methoxybenzyl) hexanediamide; (3E)-N,N,N',N'-tetrakis(2-thienylmethyl)hex-3-enediamide; N,N,N',N'-tetrakis(2-thienylmethyl)pentanediamide; N,N,N',N'-tetrakis(4-methoxybenzyl) pentanediamide; 2,2'-oxybis[N,N-bis(2-thienylmethyl)acetamide]; N,N,N',N'-tetrakis (2-thienylmethyl)octanediamide; N,N,N',N'-tetrakis(2-thienylmethyl)heptanediamide; 3-oxo-1-(2-thienyl)-2-(2-thienylmethyl)-4,7,10-trioxa-2-azadodecan-12-yl bis(2-thienylmethyl)carbamate; 2,2'-(1,3-phenylene)bis[N,N-bis(2-thienylmethyl)acetamide]; N,N,N',N'-tetrakis(4-methoxybenzyl)heptanediamide; N,N,N',N'-tetrakis(4-methoxybenzyl)succinamideethane-1,2-diyl bis[bis(2-thienylmethyl)carbamate]; N,N,N',N'-tetrakis(4-methoxybenzyl)octanediamide; N,N,N',N'-tetrakis(2-thienylmethyl) pyridine-3,5-dicarboxamide; N,N,N',N'-tetrakis(2-thienylmethyl)pyridine-2,6-dicarboxamide; N,N,N',N'-tetrakis(2-thienylmethyl)pyridine-2,4-dicarboxamide; 2,2'-(1,4-phenylene)bis [N,N-bis(2-thienylmethyl)acetamide]; N,N'-bis(4-methoxybenzyl)-N,N'-bis (2-thienylmethyl)hexanediamide; and mixtures or combinations thereof.

Also disclosed is a method of enhanced binding of integrin-expressing cells to an integrin-binding ligand which utilizes an integrin agonist compound selected from the group consisting of methyl(2S)-6-{[(benzyloxy)carbonyl]amino}-2-[benzyl(phenylsulfonyl)amino]hexanoate; methyl(2S)-6-{[(benzyloxy)carbonyl]amino}-2-[benzyl(2-thienylsulfonyl)amino]hexanoate; methyl(2S)-6-{[(benzyloxy)carbonyl]amino}-2-[(2-thienylacetyl)(2-thienylmethyl)amino]hexanoate; methyl(2S)-6-{[(benzyloxy)carbonyl]amino}-2-[(2-thienylcarbonyl)(2-thienylmethyl)amino] hexanoate; benzyl{(5S)-5-[(tert-butoxycarbonyl)amino]-6-[(2-thienylmethyl) (2-thienylsulfonyl)amino]hexyl}carbamate; benzyl{(5S)-5-[(tert-butoxycarbonyl)amino]-6-[(phenylsulfonyl)(2-thienylmethyl)amino]hexyl}carbamate; benzyl{(5S)-5-[(tert-butoxycarbonyl)amino]-6-[(2-thienylacetyl)(2-thienylmethyl)amino]hexyl}carbamate; benzyl{(5S)-5-[(tert-butoxycarbonyl)amino]-6-[(methylsulfonyl)(2-thienylmethyl)amino]hexyl} carbamate; benzyl{(5S)-5-[(tert-butoxycarbonyl)amino]-6-[(2-thienylcarbonyl)(2-thienylmethyl) amino]hexyl}carbamate; benzyl[(5S)-5-[(tert-butoxycarbonyl)amino]-6-{[(4-methoxyphenyl)sulfonyl]-(2-thienylmethyl)amino}hexyl]carbamate; benzyl{(5S)-5-[(tert-butoxycarbonyl)amino]-6-[(4-methoxybenzoyl)(2-thienylmethyl)amino]hexyl}carb amate; N,N'-heptane-1,7-diylbis[N-(2-thienylmethyl)thiophene-2-carboxamide]; N,N'-heptane-1,7-diylbis[N-(2-thienylmethyl)benzamide; N,N'-hexane-1,6-diylbis [N-(2-thienylmethyl)thiophene-2-carboxamide]; N,N'-hexane-1,6-diylbis[N-(3-methoxybenzyl) thiophene-2-carboxamide]; tert-butyl{5-[(4-methoxybenzyl)(2-thienylsulfonyl) amino]pentyl}carbamate; N-(3-methoxybenzyl)-N-{5-[(2-thienylsulfonyl)amino]pentyl} thiophene-2-sulfonamide; tert-butyl{(2S)-1,6-bis[bis(2-thienylmethyl)amino]-1,6-dioxohexan-2-yl} carbamate; tert-butyl{5-[(2-thienylcarbonyl)(2-thienylmethyl)amino]pentyl}carbamate; N-(3-methoxybenzyl)-N-{5-[(2-thienylcarbonyl)amino]pentyl}thiophene-2-carboxamide; N,N'-pentane-1,5-diylbis[N-(3-methoxybenzyl)thiophene-2-carboxamide]; and mixtures or combinations thereof.

Also disclosed is a method of enhanced binding of integrin-expressing cells to an integrin-binding ligand which utilizes an integrin agonist compound selected from the group consisting of N-{2-[bis(2-thienylmethyl)sulfamoyl]ethyl}-N-(2-thienylmethyl)thiophene-2-carboxamide; 2-{butyl[(2-thienylmethyl)carbamoyl]amino}-N,N-bis(2-thienylmethyl)ethanesulfonamide; 2-[(methylsulfonyl)(2-thienylmethyl)amino]-N,N-bis(2-thienylmethyl)ethanesulfonamide; 2-{[bis(2-thienylmethyl)carbamoyl]amino}-N,N-bis(2-thienylmethyl)ethanesulfonamide; N-{2-[bis(2-thienylmethyl)sulfamoyl]ethyl}thiophene-2-sulfonamide; N-{2-[bis(2-thienylmethyl) sulfamoyl] ethyl} -2-(2-thienyl)acetamide; N-{2-[bis(2-thienylmethyl)sulfamoyl]ethyl} thiophene-2-carboxamide; N,N-bis(2-thienylmethyl)-2-{[(2-thienylmethyl)carbamoyl]amino} ethanesulfonamide; 2-({2-[bis(2-thienylmethyl)sulfamoyl]ethyl}amino)-N,N-bis(2-thienylmethyl) acetamide; 3-[{2-[bis(2-thienylmethyl)amino]-2-oxoethyl}(butyl)amino]-N,N-bis(2-thienylmethyl) propanamide; 2-[{2-[bis(2-thienylmethyl)sulfamoyl]ethyl}(methyl)amino] -N,N-bis(2-thienylmethyl) acetamide; 3-({2-[bis(2-thienylmethyl)sulfamoyl]ethyl}amino)-N,N-bis(2-thienylmethyl) propanamide; 3-({2-[bis(2-thienylmethyl)sulfamoyl]ethyl}amino)-N,N-bis(4-methoxybenzyl) propanamide; 2-(acetyl{2-[bis(2-thienylmethyl)sulfamoyl]ethyl}amino)-N,N-bis(2-thienylmethyl) acetamide; 2-(acetyl{2-[bis(4-methoxybenzyl)sulfamoyl]ethyl}amino)-N,N-bis(2-thienylmethyl) acetamide; and mixtures or combinations thereof.

Also disclosed is a method of enhancing binding of cells to an integrin-binding ligand, wherein said agonist of integrin is a compound selected from the group consisting of tert-butyl[(2S)-1-{[bis(cyclopropylmethyl)carbamoyl]oxy}hexan-2-yl]carbamate; (2S)-[(tert-butoxycarbonyl)amino]hexyldiisobutylcarbamate; methyl(8S,12S)-12-(1,3-benzodioxol-5-yl)butyl-4-isobutyl-2-methyl-5,10-dioxo-6-oxa-4,9,11-triazatetradecan-14-oate; benzyl{(5S)-6-[bis(cyclopropylmethyl)amino]-5-[(tert-butoxycarbonyl)amino-]-6-oxohexyl} arbamate; and mixtures or combinations thereof.

### EXPERIMENTS OF THE INVENTION

### Parallel Plate Flow Detachment Assays

Detachment assays were performed as described previously. Recombinant human VCAM-1 (10 ug/mL or 5 ug/mL in 0.1 M NaHCO3 (pH 9.5)) was immobilized overnight at 4°C onto 24 H 50-mm slides cut from 15 H 100-mm polystyrene Petri dishes. The slides were washed with phosphate buffer solution (PBS), blocked with 2% (w/v) bovine serum albumin (BSA) for 2 h at room temperature, and assembled into a parallel plate flow chamber. For detachment assays, vehicle, 10 µM methyl (6S,10S)-10-(1,3-benzodioxol-5-yl)-6-butyl-3,8-dioxo-1-(2-thienyl)-2-(2-thienylmethyl)-4-oxa-2,7,9-triazadodecan-12-oate were mixed with TF-1 cells, a hematopoietic stem cell line, in low affinity running the cell carrier, and then 2.0H10⁶ cells were injected into the flow chamber and allowed to settle on the slides for 10 min. An increasing linear gradient of shear flow was pulled over the adherent cells for 300 s with the use of a computer-controlled syringe pump (Harvard Apparatus). Shear stress calculations were determined every 50 s. The shear stress in dynes/cm2 is defined as (6 µ*Q*)/(*wh²*), where µ is the viscosity of the medium (0.007); Q is the flow rate in cm³/s; w is the width of the chamber (0.3175 cm), and h is the height of the chamber (0.01524 cm). The number of cells attached was recorded by digital microscopy. Vanderslice et al have showed that a VLA-4 agonist dramatically improved the adhesion to a VCAM-1 coated surface. In reviewing these data, the applicant discovered that the compound (THI0019) stabilizes the VCAM-1 and VLA-4 interaction and upon endogenous ligand binding, the compound is displaced. The compound could be removed from an *ex vivo* cell treatment media and cells that are essentially free of the compound could be suffused into a patient. For additional details the reader is directed to Vanderslice et al The Journal of Biological Chemistry, 288, (27), p 19414B19428, 2013.

### Effective Concentrations

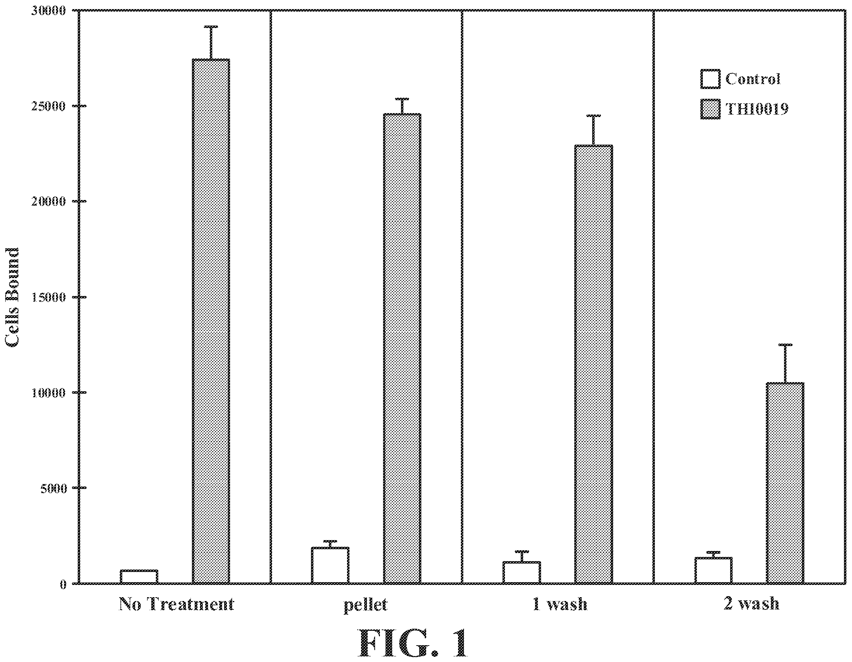

The effective concentration of a representative agonist compound, THI0019, in a cell carrier was evaluated by a static adhesion of integrin expressing cells to an immobilized integrin ligand *in vitro,* as described below. As illustrated in **Figure** 1, the effective amount of the agonist compound present in the cell carrier is at least 1 femtomolar (fM) to 10 µM. After exposure to the agonist, the resulting agonist treated cells have an enhanced ability to bind to a cognate ligand within minutes. The compound in the cell carrier was reduced from 10 µM by multiple 1000-fold serial dilutions to where cell carriers were as low as 1 fM, but the cells within the diluted carriers retained the ability to exhibit enhanced adhesion potential, in spite of the concentrations that would be deemed in effective by Vanderslice et al or PCT/US2012/066987. As such, the current art (1) expands the effective concentration range, (2) provides methods to treat and cell carrier compositions that are essentially compound free (1 fM to less than 100nM), and (3) redefines the agonist compound as NOT a classical receptor agonist, but as an inactive ingredient that is a preservative that stabilizes the integrin receptor in high affinity state, and increases the adhesive potential of the integrin expressing cell to a integrin ligand expressing cell or surface. For regulatory purposes designating the functions of the components of the carriers, this preservative action of the compound is considered as an inactive ingredient and/or an excipient.

### Method to Assess Static Adhesion

The effective concentration of THI0019, a representative agonist compound used in this example, was evaluated by a static adhesion of integrin expressing cells to an immobilized integrin ligand. The 25 amino acid alternatively spliced sequence of fibronectin named connecting segment 1 (CS1) was synthesized and conjugated to BSA. CS1-BSA at 0.3 ug/mL in 50 µL of a representative cell carrier was added to wells of a 96-well plate and allowed to coat overnight at 4°C. The representative cell carrier is isotonic Tris Buffered Saline comprising 50 mM Tris-HCl (pH 7.4), 150 mM NaCl and 1mM MgCl₂ and is a model isotonic buffer. Plates were washed with the cell carrier and blocked with 2 wt.% BSA for 1 h. 32 H 10⁶ of Jurkat cells were labeled for 30 minutes with calcein-AM (Molecular Probes), washed 2 times with the cell carrier, resuspended in 8 mL of the cell carrier, and divided equally between two tubes. One tube of the cell suspension was treated with 40 µL DMSO (control) and the other with 40 µL of 1 mM THI0019 in DMSO. The control containing tube and the THI0019 containing tube were each further divided equally among four eppendorf tubes (Tubes 1-4 for each group) such that each contained 4 H 10⁶ of the cells in 1 mL of the control or the THI0019 containing cell carrier. Tube 1 from each group was not further processed ("no treatment"). Tubes 2-4 were centrifuged at 100 x G for 5 min at RT. The supernatant was carefully removed with a pipet and each cell pellet was resuspended in 1 mL of the cell carrier without the agonist compound. Tube 2 was not further processed ("pellet"). Tubes 3-4 were again centrifuged at 100 H G for 5 min. The supernatant was carefully removed with a pipet and each cell pellet was resuspended in 1 mL of the cell carrier without the agonist compound. Tube 3 was not further processed ("1 wash"). Tube 4 was again centrifuged at 100 H G for 5 min. The supernatant was carefully removed with a pipet and the cell pellet was resuspended in 1 mL of the cell carrier without the agonist compound. Tube 4 was not further processed ("2 wash"). The final concentrations of THI0019 in the respective cell carriers for **no treatment, pellet, 1 wash,** and 2 **wash** were 10 µM, 0.01 µM,0.00001 µM,and 0.000000001 µM. 50 µL of each cell suspension was added to ligand-coated plates (2 H 10⁵ cells/well). After 30-minute incubation at 37°C, the plates were washed 3 times with binding cell carrier, the adherent cells were lysed, and fluorescence was measured on a Tecan Safire plate reader. The number of cells bound was determined by standard curves correlating fluorescence to cell number generated with the same mixtures used for the assay.

## Claims

1. A composition for use in treating a patient having a cancer or a genetic disorder treatable using hematopoietic stem cells, the composition comprising:
hematopoietic stem cells (HSCs), and
an effective amount of one or a plurality of chemical compounds of formula (I):
**R¹ - M¹ -N(R²)-M² - M³ - M⁴ - M⁵ - M⁶ - R³** (I)
where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein q is an integer from 1 to 6, and
**R³** is CON**R¹³R¹⁴**, where **R¹³** and **R¹⁴** are independently aralkyl,
wherein the one or plurality of chemical compounds are capable of enhancing integrin-mediated binding of cells to their respective ligands, and
where the effective amount is between about 1 fM and about 300 µM.

2. A composition for use in treating a patient having a cancer or a genetic disorder treatable using hematopoietic stem cells, the composition comprising:
treated hematopoietic stem cells (tHSCs) comprising:
hematopoietic stem cells (HSCs) treated with an effective amount of one or a plurality of chemical compounds of formula (I):
**R¹ - M¹ -N(R²)-M² - M³ - M⁴ - M⁵ - M⁶ - R³** (I)
where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein q is an integer from 1 to 6, and
**R³** is CON**R¹³R¹⁴**, where **R¹³** and **R¹⁴** are independently aralkyl,
wherein the one or plurality of chemical compounds are capable of enhancing integrin-mediated binding of cells to their respective ligands, where the effective amount is between about 1 fM and about 300 µM.

3. A cell carrier composition for use in treating a patient having a cancer or a genetic disorder treatable using hematopoietic stem cells, the composition comprising:
hematopoietic stem cells (HSCs), and
an effective amount of one or a plurality of chemical compounds capable of enhancing integrin-mediated binding of cells to their respective ligands,
and/or
treated hematopoietic stem cells (tHSCs) comprising:
hematopoietic stem cells (HSCs) treated with the effective amount of the one or the plurality of chemical compounds capable of enhancing integrin-mediated binding of cells to their respective ligands,
wherein the one or a plurality of chemical compounds are given by the general formula (I) :
**R¹ - M¹ -N(R²)-M² - M³ - M⁴ - M⁵ - M⁶ - R³** (I)
where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein q is an integer from 1 to 6, and
**R³** is CON**R¹³R¹⁴**, where **R¹³** and **R¹⁴** are independently aralkyl, and
where the effective amount is between about 1 fM and about 300 µM.

4. The composition for use of claim 1, claim 2 or claim 3, wherein:
the cancer or genetic disorder is selected from the group consisting of acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, and Diamond Blackfan syndrome,
the integrins targeted by these compounds are selected from the groups consisting of α4β1, α4β7, α5β1, αLβ2, αVβ3, and mixtures or combinations thereof, and
the ligands are selected from the group consisting of VCAM-1, fibronectin, MAdCAM-1, ICAM-1, ICAM-2, vitronectin, and mixtures or combinations thereof.

5. The composition for use of claim 4, wherein:
**M³** is O, and
**M⁶** is (CH₂CH₂O)_{q}, where q is an integer from 1 to 6.

6. The composition for use of claim 4, wherein:
**M³** is absent, and
**M⁶** is (CH₂)_{q}, where q is an integer from 1 to 6.

7. The composition for use of claim 1, claim 2 or claim 3, wherein the effective amount in carrier is greater than 1 fM and less than 100 nM.

8. A composition for use in enhancing homing and grafting of hematopoietic stem cells (HSCs), wherein the composition is suffused into a target tissue of a patient, where the composition comprises:
hematopoietic stem cells (HSCs) and
an effective amount of one or a plurality of chemical compounds capable of enhancing integrin-mediated binding of cells to their respective ligands, where the chemical compounds are present at an effective amount between about 1 fM and about 300 µM,
and/or
treated hematopoietic stem cells (tHSCs) comprising:
hematopoietic stem cells (HSCs) treated with the effective amount of the one or the plurality of chemical compounds capable of enhancing integrin-mediated binding of cells to their respective ligands,
wherein the one or a plurality of chemical compounds are given by the general formula (I) )
**R¹ - M¹ -N(R²)-M² - M³ - M⁴ - M⁵ - M⁶ - R³** (I)
where:
**R¹** is aryl,
**R²** is aralkyl,
**M¹** is CH₂,
**M²** is CO,
**M³** is absent or O,
**M⁴** is absent,
**M⁵** is absent,
**M⁶** is (CH₂)_{q} or (CH₂CH₂O)_{q}, wherein q is an integer from 1 to 6, and
**R³** is CON**R¹³R¹⁴**, where **R¹³** and **R¹⁴** are independently aralkyl, andwhere the target tissue is bone marrow and where the patient has a cancer or a genetic disorder treatable using hematopoietic stem cells.

9. The composition for use of claim 8, further comprising:
washing the treated hematopoietic stem cells until a concentration of the chemicals compounds is between about 1 fM and about 100 µM.

10. The composition for use of claim 8, wherein:
the cancer or genetic disorder is selected from the group consisting of acute myelogenous leukemia (AML), acute lymphoblastic leukemia (ALL), chronic myelogenous leukemia (CML), chronic lymphocytic leukemia (CLL), juvenile myelomonocytic leukemia, Hodgkin's lymphoma, non-Hodgkin's lymphoma, multiple myeloma, severe aplastic anemia, Fanconi's anemia, paroxysmal nocturnal hemoglobinuria (PNH), pure red cell aplasia, amegakaryocytosis/congenital thrombocytopenia, severe combined immunodeficiency syndrome (SCID), Wiskott-Aldrich syndrome, beta-thalassemia major, sickle cell disease, Hurler's syndrome, adrenoleukodystrophy, metachromatic leukodystrophy, myelodysplasia, refractory anemia, chronic myelomonocytic leukemia, agnogenic myeloid metaplasia, familial erythrophagocytic lymphohistiocytosis, solid tumors, chronic granulomatous disease, mucopolysaccharidoses, and Diamond Blackfan syndrome,
the integrins targeted by these compounds are selected from the groups consisting of α4β1, α4β7, α5β1, αLβ2, αVβ3, mixtures or combinations thereof, and
the ligands are selected from the groups consisting of VCAM-1, fibronectin, MAdCAM-1, ICAM-1, ICAM-2, vitronectin, and mixtures or combinations thereof.

11. The composition for use of claim 8, wherein:
**M³** is O, and
**M⁶** is (CH₂CH₂O)_{q}, where q is an integer from 1 to 6.

12. The composition for use of claim 8, wherein:
**M³** is absent, and
**M⁶** is (CH₂)_{q}, where q is an integer from 1 to 6.

13. The composition for use of any of the preceding claims, wherein **R¹M¹**, **R²**, **R¹³**, and **R¹⁴** are independently selected from the group consisting of 2-thienylmethyl, 3-methoxybenzyl, 4-methoxybenzyl, 2-(2-thienyl)ethyl, pyridin-4-ylmethyl, and pyridin-3-ylmethyl.

14. The composition for use of any of the preceding claims, wherein the effective amount is greater than 1 fM and less than 100 nM; or greater than 1 fM and less than 50 nM; or greater than 1 fM and less than 25 nM.

## Patentansprüche

1. Zusammensetzung zur Verwendung bei Behandlung eines Patienten, der einen Krebs oder eine genetische Störung aufweist, der bzw. die unter Verwendung von hämatopoietischen Stammzellen behandelbar ist, wobei die Zusammensetzung umfasst:
hämatopoietische Stammzellen (HSCs), und
eine wirksame Menge von einer oder einer Mehrzahl von chemischen Verbindungen der Formel (I):
**R¹ - M¹ - N(R²) - M² - M³ - M⁴ - M⁵ - M⁶ - R³** (I)
worin:
**R¹** Aryl ist,
**R²** Aralkyl ist,
**M¹** CH₂ ist,
**M²** CO ist,
**M³** nicht vorhanden oder O ist,
**M⁴** nicht vorhanden ist,
**M⁵** nicht vorhanden ist,
**M⁶** (CH₂)_{q} oder (CH₂CH₂O)q ist, wobei q eine ganze Zahl von 1 bis 6 ist, und
**R³** CON**R¹³R¹⁴** ist, worin **R¹³** und **R¹⁴** unabhängig Aralkyl sind,
wobei die eine oder die Mehrzahl von chemischen Verbindungen fähig sind, Integrin-vermittelte Bindung von Zellen an deren jeweilige Liganden zu verbessern, und
wobei die wirksame Menge zwischen ungefähr 1 fM und ungefähr 300 µM liegt.

2. Zusammensetzung zur Verwendung bei Behandlung eines Patienten, der einen Krebs oder eine genetische Störung aufweist, der bzw. die unter Verwendung von hämatopoietischen Stammzellen behandelbar ist, wobei die Zusammensetzung umfasst:
behandelte hämatopoietische Stammzellen (tHSCs), umfassend:
hämatopoietische Stammzellen (HSCs), behandelt mit einer wirksamen Menge von einer oder einer Mehrzahl von chemischen Verbindungen der Formel (I):
**R¹ - M¹ - N(R²) - M² - M³ - M⁴ - M⁵ - M⁶ - R³** (I)
worin:
**R¹** Aryl ist,
**R²** Aralkyl ist,
**M¹** CH₂ ist,
**M²** CO ist,
**M³** nicht vorhanden oder O ist,
**M⁴** nicht vorhanden ist,
**M⁵** nicht vorhanden ist,
**M⁶** (CH₂)_{q} oder (CH₂CH₂O)q ist, wobei q eine ganze Zahl von 1 bis 6 ist, und
**R³** CON**R¹³R¹⁴** ist, worin **R¹³** und **R¹⁴** unabhängig Aralkyl sind,
wobei die eine oder die Mehrzahl von chemischen Verbindungen fähig sind, Integrin-vermittelte Bindung von Zellen an deren jeweilige Liganden zu verbessern, wobei die wirksame Menge zwischen ungefähr 1 fM und ungefähr 300 µM liegt.

3. Zellträgerzusammensetzung zur Verwendung bei Behandlung eines Patienten, der einen Krebs oder eine genetische Störung aufweist, der bzw. die unter Verwendung von hämatopoietischen Stammzellen behandelbar ist, wobei die Zusammensetzung umfasst:
hämatopoietische Stammzellen (HSCs), und
eine wirksame Menge von einer oder einer Mehrzahl von chemischen Verbindungen mit der Fähigkeit, Integrin-vermittelte Bindung von Zellen an deren jeweilige Liganden zu verbessern,
und/oder
behandelte hämatopoietische Stammzellen (tHSCs), umfassend:
hämatopoietische Stammzellen (HSCs), behandelt mit der wirksamen Menge der einen oder der Mehrzahl von chemischen Verbindungen mit der Fähigkeit, Integrin-vermittelte Bindung von Zellen an deren jeweilige Liganden zu verbessern,
wobei die eine oder die Mehrzahl von chemischen Verbindungen durch die allgemeine Formel (I) angegeben sind:
**R¹ - M¹ - N(R²) - M² - M³ - M⁴ - M⁵ - M⁶ - R³** (I)
worin:
**R¹** Aryl ist,
**R²** Aralkyl ist,
**M¹** CH₂ ist,
**M²** CO ist,
**M³** nicht vorhanden oder O ist,
**M⁴** nicht vorhanden ist,
**M⁵** nicht vorhanden ist,
**M⁶** (CH₂)_{q} oder (CH₂CH₂O)q ist, wobei q eine ganze Zahl von 1 bis 6 ist, und
**R³** CON**R¹³R¹⁴** ist, worin **R¹³** und **R¹⁴** unabhängig Aralkyl sind, und
wobei die wirksame Menge zwischen ungefähr 1 fM und ungefähr 300 µM liegt.

4. Zusammensetzung zur Verwendung nach Anspruch 1, Anspruch 2 oder Anspruch 3, wobei:
der Krebs oder die genetische Störung aus der Gruppe ausgewählt ist, die besteht aus akuter myeloischer Leukämie (AML), akuter lymphoblastischer Leukämie (ALL), chronischer myeloischer Leukämie (CML), chronischer lymphatischer Leukämie (CLL), juveniler myelomonozytärer Leukämie, Hodgkin-Lymphom, Non-Hodgkin-Lymphom, multiplem Myelom, schwerer aplastischer Anämie, Fanconi-Anämie, paroxysmaler nächtlicher Hämoglobinurie (PNH), Aplasie der roten Blutkörperchen, Amegakaryozytose/angeborener Thrombozytopenie, Syndrom des schweren kombinierten Immundefekts (SCID), Wiskott-Aldrich-Syndrom, Beta-Thalassaemia major, Sichelzellkrankheit, Hurler-Syndrom, Adrenoleukodystrophie, metachromatischer Leukodystrophie, Myelodysplasie, refraktärer Anämie, chronischer myelomonozytärer Leukämie, agnogener myeloischer Metaplasie, familiärer erythrophagozytischer Lymphohistiozytose, soliden Tumoren, chronischer granulomatöser Erkrankung, Mukopolysaccharidosen und Diamond-Blackfan-Syndrom,
die durch diese Verbindungen angezielten Integrine aus den Gruppen ausgewählt sind, die aus α4β1, α4β7, α5β1, αLβ2, αVβ3, und Mischungen oder Kombinationen davon, bestehen, und
die Liganden aus der Gruppe ausgewählt sind, die aus VCAM-1, Fibronektin, MAdCAM-1, ICAM-1, ICAM-2, Vitronektin, und Mischungen oder Kombinationen davon, besteht.

5. Zusammensetzung zur Verwendung nach Anspruch 4, wobei:
**M³** O ist, und
**M⁶** (CH₂CH₂O)_{q} ist, worin q eine ganze Zahl von 1 bis 6 ist.

6. Zusammensetzung zur Verwendung nach Anspruch 4, wobei:
**M³** nicht vorhanden ist, und
**M⁶** (CH₂)_{q} ist, worin q eine ganze Zahl von 1 bis 6 ist.

7. Zusammensetzung zur Verwendung nach Anspruch 1, Anspruch 2 oder Anspruch 3, wobei die wirksame Menge im Träger größer als 1 fM und kleiner als 100 nM ist.

8. Zusammensetzung zur Verwendung bei Verbesserung von Homing und Grafting hämatopoietischer Stammzellen (HSCs), wobei die Zusammensetzung in ein Zielgewebe eines Patienten suffusionsartig verteilt wird, wobei die Zusammensetzung umfasst:
hämatopoietische Stammzellen (HSCs), und
eine wirksame Menge von einer oder einer Mehrzahl von chemischen Verbindungen mit der Fähigkeit, Integrin-vermittelte Bindung von Zellen an deren jeweilige Liganden zu verbessern,
wobei die chemischen Verbindungen in einer wirksamen Menge zwischen ungefähr 1 fM und ungefähr 300 µM vorliegen,
und/oder
behandelte hämatopoietische Stammzellen (tHSCs), umfassend:
hämatopoietische Stammzellen (HSCs), behandelt mit der wirksamen Menge der einen oder der Vielzahl von chemischen Verbindungen mit der Fähigkeit, Integrin-vermittelte Bindung von Zellen an deren jeweilige Liganden zu verbessern,
wobei die eine oder eine Vielzahl von chemischen Verbindungen durch die allgemeine Formel (I) angegeben sind:
**R¹ - M¹ - N(R²) - M² - M³ - M⁴ - M⁵ - M⁶ - R³** (I)
worin:
**R¹** Aryl ist,
**R²** Aralkyl ist,
**M¹** CH₂ ist,
**M²** CO ist,
**M³** nicht vorhanden oder O ist,
**M⁴** nicht vorhanden ist,
**M⁵** nicht vorhanden ist,
**M⁶** (CH₂)_{q} oder (CH₂CH₂O)q ist, wobei q eine ganze Zahl von 1 bis 6 ist, und
**R³** CON**R¹³R¹⁴** ist, worin **R¹³** und **R¹⁴** unabhängig Aralkyl sind, und worin das Zielgewebe Knochenmark ist und worin der Patient einen Krebs oder eine genetische Störung aufweist, der bzw. die unter Verwendung von hämatopoietischen Stammzellen behandelbar ist.

9. Zusammensetzung zur Verwendung nach Anspruch 8, weiterhin umfassend:
Waschen der behandelten hämatopoietischen Stammzellen, bis eine Konzentration der chemischen Verbindungen zwischen ungefähr 1 fM und ungefähr 100 µM liegt.

10. Zusammensetzung zur Verwendung nach Anspruch 8, wobei:
der Krebs oder die genetische Störung aus der Gruppe ausgewählt ist, die besteht aus akuter myeloischer Leukämie (AML), akuter lymphoblastischer Leukämie (ALL), chronischer myeloischer Leukämie (CML), chronischer lymphatischer Leukämie (CLL), juveniler myelomonozytärer Leukämie, Hodgkin-Lymphom, Non-Hodgkin-Lymphom, multiplem Myelom, schwerer aplastischer Anämie, Fanconi-Anämie, paroxysmaler nächtlicher Hämoglobinurie (PNH), Aplasie der roten Blutkörperchen, Amegakaryozytose/angeborener Thrombozytopenie, Syndrom des schweren kombinierten Immundefekts (SCID), Wiskott-Aldrich-Syndrom, Beta-Thalassaemia major, Sichelzellkrankheit, Hurler-Syndrom, Adrenoleukodystrophie, metachromatischer Leukodystrophie, Myelodysplasie, refraktärer Anämie, chronischer myelomonozytärer Leukämie, agnogener myeloischer Metaplasie, familiärer erythrophagozytischer Lymphohistiozytose, soliden Tumoren, chronischer granulomatöser Erkrankung, Mukopolysaccharidosen und Diamond-Blackfan-Syndrom,
die durch diese Verbindungen angezielten Integrine aus den Gruppen ausgewählt sind, die aus α4β1, α4β7, α5β1, αLβ2, αVβ3, Mischungen oder Kombinationen davon bestehen, und
die Liganden aus den Gruppen ausgewählt sind, die aus VCAM-1, Fibronektin, MAdCAM-1, ICAM-1, ICAM-2, Vitronektin, und Mischungen oder Kombinationen davon, bestehen.

11. Zusammensetzung zur Verwendung nach Anspruch 8, wobei:
**M³** O ist, und
**M⁶** (CH₂CH₂O)_{q} ist, worin q eine ganze Zahl von 1 bis 6 ist.

12. Zusammensetzung zur Verwendung nach Anspruch 8, wobei:
**M³** nicht vorhanden ist, und
**M⁶** (CH₂)_{q} ist, worin q eine ganze Zahl von 1 bis 6 ist.

13. Zusammensetzung zur Verwendung nach einem der vorstehenden Ansprüche, wobei **R¹M¹**, **R², R¹³** und **R¹⁴** unabhängig aus der Gruppe ausgewählt sind, die besteht aus 2-Thienylmethyl, 3-Methoxybenzyl, 4-Methoxybenzyl, 2-(2-Thienyl)ethyl, Pyridin-4-ylmethyl und Pyridin-3-ylmethyl.

14. Zusammensetzung zur Verwendung nach einem der vorstehenden Ansprüche, wobei die wirksame Menge größer als 1 fM und kleiner als 100 nM ist; oder größer als 1 fM und kleiner als 50 nM; oder größer als 1 fM und kleiner als 25 nM.

## Revendications

1. Composition pour utilisation dans le traitement d'un patient ayant un cancer ou un trouble génétique traitable avec des cellules souches hématopoïétiques, la composition comprenant :
des cellules souches hématopoïétiques (HSC), et
une quantité efficace d'un ou d'une pluralité de composés chimiques de formule (I) :
**R¹ - M¹ -N(R²)-M² - M³ - M⁴ - M⁵ - M⁶ - R³** (I)
où :
**R¹** est un aryle,
**R²** est un aralkyle,
**M¹** est CH₂,
**M²** est CO,
**M³** est absent ou O,
**M⁴** est absent,
**M⁵** est absent,
**M⁶** est (CH₂)_{q} ou (CH₂CH₂O)_{q}, dans laquelle q est un entier de 1 à 6, et
**R³** est CON**R¹³R¹⁴**, où **R¹³** et **R¹⁴** sont indépendamment un aralkyle,
dans laquelle l'un ou la pluralité de composés chimiques sont capables d'améliorer la liaison médiée par l'intégrine de cellules à leurs ligands respectifs, et
dans laquelle la quantité efficace est d'entre environ 1 fM et environ 300 µM.

2. Composition pour utilisation dans le traitement d'un patient ayant un cancer ou un trouble génétique traitable avec des cellules souches hématopoïétiques, la composition comprenant :
des cellules souches hématopoïétiques traitées (tHSC) comprenant :
des cellules souches hématopoïétiques (HSC) traitées avec une quantité efficace d'un ou d'une pluralité de composés chimiques de formule (I) :
**R¹** - **M¹ -N(R²)-M² - M³ - M⁴ - M⁵ - M⁶ - R³** (I)
où :
**R¹** est un aryle,
**R²** est un aralkyle,
**M¹** est CH₂,
**M²** est CO,
**M³** est absent ou O,
**M⁴** est absent,
**M⁵** est absent,
**M⁶** est (CH₂)_{q} ou (CH₂CH₂O)_{q}, dans laquelle q est un entier de 1 à 6, et
**R³** est CON**R¹³R¹⁴**, où **R¹³** et **R¹⁴** sont indépendamment un aralkyle,
dans laquelle l'un ou la pluralité de composés chimiques sont capables d'améliorer la liaison médiée par l'intégrine de cellules à leurs ligands respectifs, dans laquelle la quantité efficace est d'entre environ 1 fM et environ 300 µM.

3. Composition porteuse de cellules pour utilisation dans le traitement d'un patient ayant un cancer ou un trouble génétique traitable avec des cellules souches hématopoïétiques, la composition comprenant :
des cellules souches hématopoïétiques (HSC), et
une quantité efficace d'un ou d'une pluralité de composés chimiques capables d'améliorer la liaison médiée par l'intégrine de cellules à leurs ligands respectifs,
et/ou
des cellules souches hématopoïétiques traitées (tHSC) comprenant :
des cellules souches hématopoïétiques (HSC) traitées avec une quantité efficace de l'un ou de la pluralité de composés chimiques capables d'améliorer la liaison médiée par l'intégrine de cellules à leurs ligands respectifs,
dans laquelle l'un ou une pluralité de composés chimiques sont donnés par la formule générale (I) :
**R¹ - M¹ -N(R²)-M² - M³ - M⁴ - M⁵ - M⁶ - R³** (I)
où :
**R¹** est un aryle,
**R²** est un aralkyle,
**M¹** est CH₂,
**M²** est CO,
**M³** est absent ou O,
**M⁴** est absent,
**M⁵** est absent,
**M⁶** est (CH₂)_{q} ou (CH₂CH₂O)_{q}, dans laquelle q est un entier de 1 à 6, et
**R³** est CON**R¹³R¹⁴**, où **R¹³** et **R¹⁴** sont indépendamment un aralkyle, et
dans laquelle la quantité efficace est d'entre environ 1 fM et environ 300 µM.

4. Composition pour utilisation selon la revendication 1, la revendication 2 ou la revendication 3, dans laquelle :
le cancer ou trouble génétique est sélectionné parmi le groupe constitué de : leucémie myéloïde aiguë (LMA), leucémie lymphoblastique aiguë (LLA), leucémie myéloïde chronique (LMC), leucémie lymphocytaire chronique (LLC), leucémie myélomonocytaire juvénile, lymphome de Hodgkin, lymphome non hodgkinien, myélome multiple, anémie aplasique grave, anémie de Fanconi, hémoglobinurie paroxystique nocturne (HPN), aplasie pure des globules rouges, amégakaryocytose/thrombocytopénie congénitale, syndrome de déficit immunitaire combiné sévère (SCID), syndrome de Wiskott-Aldrich, bêta thalassémie majeure, anémie falciforme, syndrome de Hurler, adrénoleucodystrophie, leucodystrophie métachromatique, myélodysplasie, anémie réfractaire, leucémie myélomonocytaire chronique, métaplasie myéloïde agnogénique, lymphohistiocytose érythrophagocytaire familiale, tumeurs solides, maladie granulomateuse chronique, mucopolysaccharidoses et syndrome de Diamond Blackfan,
les intégrines ciblées par ces composés sont sélectionnées parmi les groupes constitués de α4β1, α4β7, α5β1, αLβ2, αVβ3, et de mélanges ou de combinaisons de celles-ci, et
les ligands sont sélectionnés parmi le groupe constitué de VCAM-1, fibronectine, MAdCAM-1, ICAM-1, ICAM-2, vitronectine, et de mélanges ou de combinaisons de ceux-ci.

5. Composition pour utilisation selon la revendication 4, dans laquelle :
**M³** est O, et
**M⁶** est (CH₂CH₂O)_{q}, où q est un entier de 1 à 6.

6. Composition pour utilisation selon la revendication 4, dans laquelle :
**M³** est absent, et
**M⁶** est (CH₂)_{q}, où q est un entier de 1 à 6.

7. Composition pour utilisation selon la revendication 1, la revendication 2 ou la revendication 3, dans laquelle la quantité efficace dans la porteuse est supérieure à 1 fM et inférieure à 100 nM.

8. Composition pour utilisation dans l'amélioration de l'hébergement et de la greffe de cellules souches hématopoïétiques (HSC), où la composition est répandue dans un tissu cible d'un patient, où la composition comprend :
des cellules souches hématopoïétiques (HSC), et
une quantité efficace d'un ou d'une pluralité de composés chimiques capables d'améliorer la liaison médiée par l'intégrine de cellules à leurs ligands respectifs,
dans laquelle les composés chimiques sont présents en une quantité efficace d'entre environ 1 fM et environ 300 µM,
et/ou
des cellules souches hématopoïétiques traitées (tHSC) comprenant :
des cellules souches hématopoïétiques (HSC) traitées avec une quantité efficace de l'un ou de la pluralité de composés chimiques capables d'améliorer la liaison médiée par l'intégrine de cellules à leurs ligands respectifs,
dans laquelle l'un ou une pluralité de composés chimiques sont donnés par la formule générale (I) :
**R¹** - **M¹ -N(R²)-M² - M³ - M⁴ - M⁵ - M⁶ - R³** (I)
où :
**R¹** est un aryle,
**R²** est un aralkyle,
**M¹** est CH₂,
**M²** est CO,
**M³** est absent ou O,
**M⁴** est absent,
**M⁵** est absent,
**M⁶** est (CH₂)_{q} ou (CH₂CH₂O)_{q}, dans laquelle q est un entier de 1 à 6, et
**R³** est CON**R¹³R¹⁴**, où **R¹³** et **R¹⁴** sont indépendamment un aralkyle, et
où le tissu cible est de la moelle osseuse et où le patient a un cancer ou un trouble génétique traitable avec des cellules souches hématopoïétiques.

9. Composition pour utilisation selon la revendication 8, comprenant en outre l'étape consistant à :
laver les cellules souches hématopoïétiques traitées jusqu'à ce qu'une concentration des composés chimiques soit d'entre environ 1 fM et environ 100 µM

10. Composition pour utilisation selon la revendication 8, où :
le cancer ou trouble génétique est sélectionné parmi le groupe constitué de : leucémie myéloïde aiguë (LMA), leucémie lymphoblastique aiguë (LLA), leucémie myéloïde chronique (LMC), leucémie lymphocytaire chronique (LLC), leucémie myélomonocytaire juvénile, lymphome de Hodgkin, lymphome non hodgkinien, myélome multiple, anémie aplasique grave, anémie de Fanconi, hémoglobinurie paroxystique nocturne (HPN), aplasie pure des globules rouges, amégakaryocytose/thrombocytopénie congénitale, syndrome de déficit immunitaire combiné sévère (SCID), syndrome de Wiskott-Aldrich, bêta thalassémie majeure, anémie falciforme, syndrome de Hurler, adrénoleucodystrophie, leucodystrophie métachromatique, myélodysplasie, anémie réfractaire, leucémie myélomonocytaire chronique, métaplasie myéloïde agnogénique, lymphohistiocytose érythrophagocytaire familiale, tumeurs solides, maladie granulomateuse chronique, mucopolysaccharidoses et syndrome de Diamond Blackfan,
les intégrines ciblées par ces composés sont sélectionnées parmi les groupes constitués de α4β1, α4β7, α5β1, αLβ2, αVβ3, de mélanges ou de combinaisons de celles-ci, et
les ligands sont sélectionnés parmi les groupes constitués de VCAM-1, fibronectine, MAdCAM-1, ICAM-1, ICAM-2, vitronectine, et de mélanges ou de combinaisons de ceux-ci.

11. Composition pour utilisation selon la revendication 8, dans laquelle :
**M³** est O, et
**M⁶** est (CH₂CH₂O)_{q}, où q est un entier de 1 à 6.

12. Composition pour utilisation selon la revendication 8, dans laquelle :
**M³** est absent, et
**M⁶** est (CH₂)_{q}, où q est un entier de 1 à 6.

13. Composition pour utilisation selon l'une quelconque des revendications précédentes, dans laquelle **R¹M¹**, **R²**, **R¹³** et **R¹⁴** sont sélectionnés indépendamment parmi le groupe constitué de 2-thiénylméthyle, 3-méthoxybenzyle, 4-méthoxybenzyle, 2-(2-thiényl)éthyle, pyridin-4-ylméthyle et pyridin-3-ylméthyle.

14. Composition pour utilisation selon l'une quelconque des revendications précédentes, dans laquelle la quantité efficace est supérieure à 1 fM et inférieure à 100 nM ; ou supérieure à 1 fM et inférieure à 50 nM ; ou supérieure à 1 fM et inférieure à 25 nM.
